(19) Europäisches Patentamt
European Patent Office
Office européen des brevets

(11) Veröffentlichungsnummer: **0 302 312 A2**

(12) **EUROPÄISCHE PATENTANMELDUNG**

(21) Anmeldenummer: **88111770.9**

(22) Anmeldetag: **21.07.88**

(51) Int. Cl.4: **C07D 239/47 , C07D 405/12 , C07D 403/12 , A01N 43/54**

(30) Priorität: **03.08.87 DE 3725638**

(43) Veröffentlichungstag der Anmeldung: **08.02.89 Patentblatt 89/06**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Sasse, Klaus, Dr.**
**Pützweg 13**
**D-5060 Bergisch-Gladbach 2(DE)**
Erfinder: **Fischer, Reiner, Dr.**
**Rembrandtstrasse 15**
**D-4019 Monheim(DE)**
Erfinder: **Schwamborn, Michael, Dr.**
**von-Lohe-Strasse 9**
**D-5000 Köln 80(DE)**
Erfinder: **Krebs, Andreas, Dr.**
**Im Gartenfeld 70**
**D-5068 Odenthal-Holz(DE)**
Erfinder: **Hagemann, Hermann, Dr.**
**Kandinsky-Strasse 52**
**D-5090 Leverkusen 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Waldwinkel 110**
**D-5060 Bergisch-Gladbach 2(DE)**
Erfinder: **Strang, Harry, Dr.**
**Unterdorfstrasse 6A**
**D-4000 Düsseldorf 31(DE)**

(54) **Neue Aryloxy (bzw. thio)-aminopyrimidine.**

(57) Die Erfindung betrifft neue Aryloxy (bzw. thio(aminopyrimidine der Formel (I)

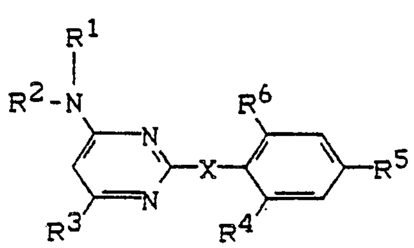

(I)

EP 0 302 312 A2

in welcher
$R^1$, $R^2$, $R^3$, $R^4$, $R^5$, $R^6$ und X die in der Beschreibung angegebene Bedeutung haben, mehrere Verfahren sowie neue Zwischenprodukte zu ihrer Herstellung und ihre Verwendung als Herbizide.

## Neue Aryloxy (bzw. thio)aminopyrimidine

Die vorliegende Erfindung betrifft neue substituierte Aryloxy-(bzw. thio)aminopyrimidinderivate, ihre Herstellung, Zwischenprodukte zu ihrer Herstellung und ihre Verwendung als Herbizide.

Es ist bereits bekannt, daß bestimmte Diaminopyrimidinderivate herbizide Eigenschaften besitzen (vgl. R. Wegler "Chemie der Pflanzenschutz- und Schädlingsbekämpfungsmittel" Bd. V, Seite 318-319, 1977). Ferner ist bekannt, daß bestimmte Pyrimidyloxy (bzw. thio)phenylderivate (vgl. JA 9474/1967, DE-OS 3 442 077) herbizide Wirkung zeigen.

Ihre Wirkung ist jedoch unter bestimmten Bedingungen, z.B. bei niedrigen Auifwandmengen nicht immer befriedigend. Ebenso ist die Selektivität nicht immer ausreichend.

Ebenso ist bekannt, daß bestimmte 4-Aminopyrimidinderivate fungizide Eigenschaften aufweisen (DE-OS 3 504 895).

Weitere monoaminosubstituierte Pyrimidine lassen ebenfalls herbizide Wirkungen erkennen (vgl. EP-A 1 187). So zeigt 2-(3-Chlorphenoxy)-4-dimethylaminopyrimidin herbizide Wirkung, die jedoch nicht immer zufriedenstellend ist.

Es wurden nun neue Aryloxy (bzw. thio)aminopyrimidine der Formel (I) gefunden,

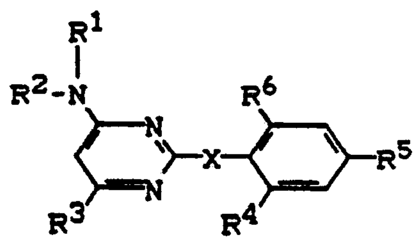

(I)

in welcher

$R^1$ für Wasserstoff, Alkyl, Alkoxyalkyl, Cycloalkyl oder Alkenyl steht,

$R^2$ für Wasserstoff oder Alkyl steht oder

$R^1$ und $R^2$ gemeinsam mit dem Stickstoffatom, an das sie gebunden sind, auch einen drei- bis sechsgliedrigen Ring mit 1 oder 2 weiteren Heteroatomen bilden können,

$R^3$ für Wasserstoff oder gegebenenfalls substituiertes Alkyl steht,

X für Sauerstoff oder Schwefel steht,

$R^4$ und $R^6$ unabhängig voneinander für Wasserstoff, Halogen, Nitro oder für jeweils gegebenenfalls substituiertes Alkyl oder Alkoxy stehen und

$R^5$ für Wasserstoff, Halogen, Nitro, Hydroxy, jeweils gegebenenfalls substituiertes Alkyl, Alkenyl oder Alkinyl, jeweils gegebenenfalls substituiertes Alkoxy, Alkoxyalkyloxy, Alkoxyalkoxyalkyloxy, Alkylthio, Alkylsulfoxy oder Alkylsulfonyl, jeweils gegebenenfalls substituiertes Alkylcarbonyl oder Alkoxycarbonylalkyl steht oder

$R^5$ einen Rest der Formel

$-NR^7R^8$

steht, wobei

$R^7$ für Wasserstoff, gegebenenfalls substituiertes Alkyl, für Alkenyl oder Alkinyl steht und

$R^8$ für Wasserstoff, Alkyl oder einen Rest der Formel

$$-\underset{\underset{B}{\|}}{C}-R^9$$

steht, in welcher

B für Sauerstoff oder Schwefel steht und

$R^9$ für Alkyl, Alkenyl oder Alkinyl steht, welche gegebenenfalls durch Halogen, Nitro, Cyano, gegebenenfalls substituiertes Aryl, und/oder durch einen oder mehrere Reste der Formel

$-C-R^{10}$

substituiert sind, wobei
D für Sauerstoff, Schwefel, Sulfoxy oder Sulfonyl steht und
$R^{10}$ für Wasserstoff, jeweils gegebenenfalls substituiertes Alkyl, Alkenyl oder Aryl steht, oder
$R^9$ weiterhin für gegebenenfalls substituiertes Cycloalkyl oder einen gegebenenfalls substituierten Heterocyclus steht,
$R^5$ weiterhin für einen Rest der Formeln

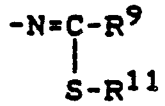

steht wobei
$R^8$ die oben angegebene Bedeutung hat und
$R^{11}$ für gegebenenfalls substituiertes Alkyl, für Alkoxyalkyl, Alkenyl oder Alkinyl steht,
$R^5$ weiterhin für einen Rest der Formel

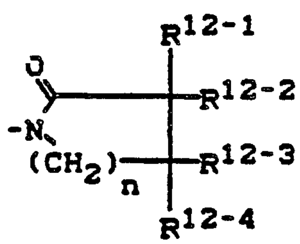

steht, wobei
n für die Zahl 1 oder 2 steht und
$R^{12-1}$, $R^{12-2}$, $R^{12-3}$ und $R^{12-4}$ unabhängig voneinander für Wasserstoff, Halogen oder Alkyl stehen,
$R^5$ weiterhin für einen Rest der Formel

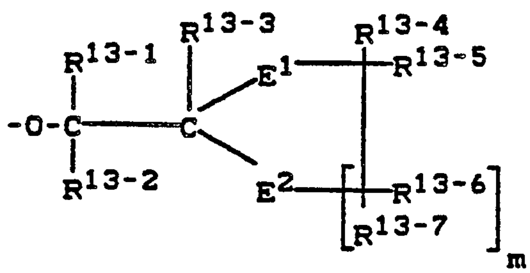

steht, wobei
$E^1$ und $E^2$ unabhängig voneinander für Sauerstoff oder Schwefel stehen und
$R^{13-1}$ bis $R^{13-7}$ unabhängig voneinander für Wasserstoff oder Alkyl stehen und
m für die Zahl 1 oder 2 steht.

Weiterhin wurde gefunden, daß man die Aryloxy (bzw. thio)aminopyrimidine der Formel (I) erhält, wenn man

A) Pyrimidinderivate der Formel (II),

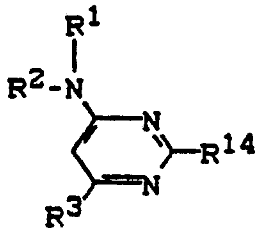

(II)

in welcher
$R^1$, $R^2$ und $R^3$ die oben angegebene Bedeutung haben und
$R^{14}$ für Halogen oder Alkylsulfonyl steht,
mit (Thio)phenolen der Formel (III)

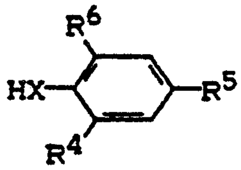

(III)

in welcher
$R^4$, $R^5$, $R^6$ und X die angegebene Bedeutung haben,
gegebenenfalls in Gegenwart eines Verdünnungsmittels und gegebenenfalls in Gegenwart eines Säurebindemittels umsetzt, oder

B) Halogenpyrimidinderivate der allgemeinen Formel (IV)

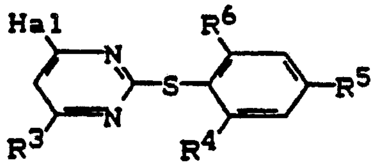

(IV)

in welcher
$R^3$, $R^4$, $R^5$ und $R^6$ die oben angegebene Bedeutung haben mit der Maßgabe, daß mindestens einer der Reste $R^4$, $R^5$ oder $R^6$ starke Elektronenakzeptoreigenschaft besitzt wie z.B. Nitro oder Halogenalkyl und
Hal für Halogen steht,
mit einem Amin der allgemeinen Formel (V)

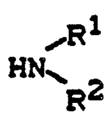

(V)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt oder

C-$C_1$) Aryloxy (bzw. thio)aminopyrimidine der Formel (Ia)

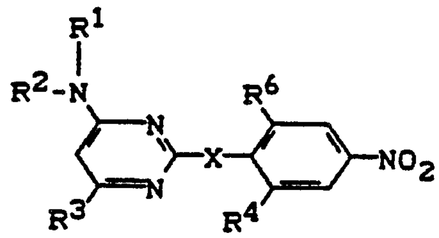

(Ia)

in welcher
$R^1$, $R^2$, $R^3$, $R^4$, $R^6$ und X die oben angegebene Bedeutung haben,
mit Reduktionsmitteln gegebenenfalls in Gegenwart eines Verdünnungsmittels und gegebenenfalls in Gegenwart eines Katalysators umsetzt und

C-$C_2$) die nach Verfahren C-$C_1$ erhaltenen Aryloxy (bzw. thio)aminopyrimidine der Formel (Ib)

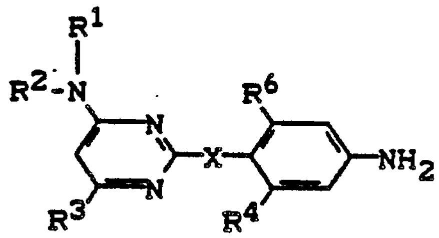

(Ib)

in welcher
$R^1$, $R^2$, $R^3$, $R^4$, $R^6$ und X die oben angegebene Bedeutung haben,
α) mit Säurehalogeniden der Formel (VI),

$$Hal-\overset{\overset{O}{\|}}{C}-R^9 \qquad (VI)$$

in welcher
$R^9$ die oben angegebene Bedeutung hat und
Hal für Halogen, insbesondere Chlor oder Brom steht,
gegebenenfalls in Gegenwart eines Verdünnungsmittels und gegebenenfalls in Gegenwart eines Säurebindemittels umsetzt,
oder
β) mit symmetrischen Carbonsäureanhydriden der allgemeinen Formel (VII),

$$R^9-\overset{\overset{O}{\|}}{C}-O-\overset{\overset{O}{\|}}{C}-R^9 \qquad (VII)$$

in welcher
$R^9$ die oben angegebene Bedeutung hat,
gegebenenfalls in Gegenwart eines Verdünnungsmittels umsetzt,
oder
γ) mit asymmetrischen Säureanhydriden der Formel (VIII),

$$R-O-\overset{\overset{O}{\|}}{C}-O-\overset{\overset{O}{\|}}{C}-R^9 \qquad (VIII)$$

in welcher
$R^9$ die oben angegebene Bedeutung hat und
R für Alkyl oder Alkylsulfonyl mit jeweils 1 bis 4 Kohlenstoffatomen, Phenyl oder Phenylsulfonyl steht,

gegebenenfalls in Gegenwart eines Verdünnungsmittels umsetzt,
oder
δ) mit ω-Halogencarbonsäurehalogeniden der Formel (IX),

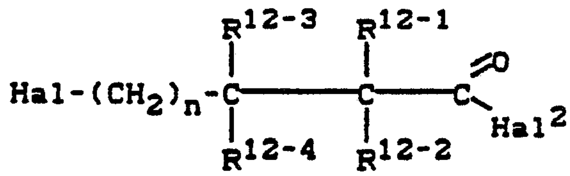

(IX)

in welcher
$R^{12-1}$, $R^{12-2}$, $R^{12-3}$, $R^{12-4}$ und n die oben angegebene Bedeutung haben und
Hal und $Hal^2$ für Halogen stehen,
gegebenenfalls in Gegenwart eines Säurebindemittels und gegebenenfalls in Gegenwart eines Verdünnungsmittels in einer zweistufigen Reaktionsfolge umsetzt,
oder

D) Aryloxy (bzw. thio)aminopyrimidine der Formel (Ic)

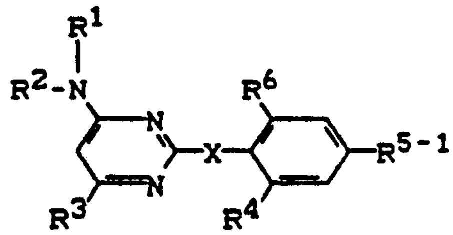

(Ic)

in welcher
$R^1$, $R^2$, $R^3$, $R^4$, $R^6$ und X die oben angegebene Bedeutung haben, und
$R^{5-1}$ für den Rest

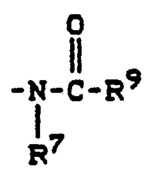

steht, wobei $R^7$ und $R^9$ die oben angegebene Bedeutung haben,
mit Schwefelungsreagenzien, wie beispielsweise Phosphor-V-sulfid oder 2,4-Bis-(4-methoxphenyl)-2,4-dithiono-1,2,3,4-dithia-phosphetan (Lawesson-Reagenz) umsetzt zu Verbindungen der Formel (Id)

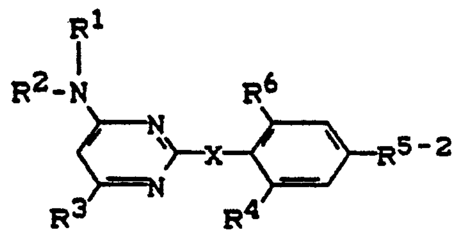

(Id)

in welcher
$R^1$, $R^2$, $R^3$, $R^4$, $R^6$ und X die oben angegebene Bedeutung haben, und
$R^{5-2}$ für den Rest

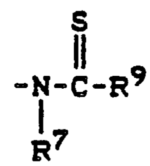

steht, wobei $R^7$ und $R^9$ die oben angegebene Bedeutung haben,
oder

E) die nach dem Verfahren D) hergestellten Verbindungen (Id), in denen $R^7$ für Wasserstoff steht, mit Verbindungen der Formel (X)

$L-R^{11}$ (X)

in welcher
$R^{11}$ die oben angegebene Bedeutung hat und
L für Halogen, Alkylsulfonyloxy oder gegebenenfalls substituiertes Phenylsulfonyloxy steht,
mit deprotonierenden Basen und gegebenenfalls in Gegenwart eines Verdünnungsmittels zu Verbindungen der allgemeinen Formel (Ie) umsetzt

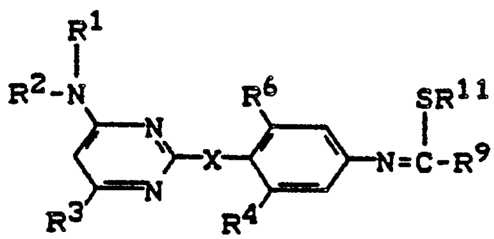

(Ie)

in welcher
$R^1$, $R^2$, $R^3$, $R^4$, $R^6$, $R^9$, $R^{11}$ und X die oben angegebene Bedeutung haben,
oder

F) Aryloxy (bzw. thio)aminopyrimidine der Formel (If)

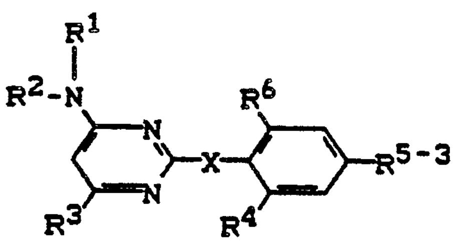

(If)

in welcher
$R^1$, $R^2$, $R^3$, $R^4$, $R^6$ und X die oben angegebene Bedeutung haben, und
$R^{5-3}$ für Hydroxy steht,
mit Verbindungen der allgemeinen Formel (XI),

$R^{5-4}$-G (XI)

in welcher
$R^{5-4}$ für jeweils gegebenenfalls substituiertes Alkyl, Alkenyl, Alkoxyalkyl, Alkylthioalkyl oder Alkylcarbonylalkyl oder für einen Rest der Formel

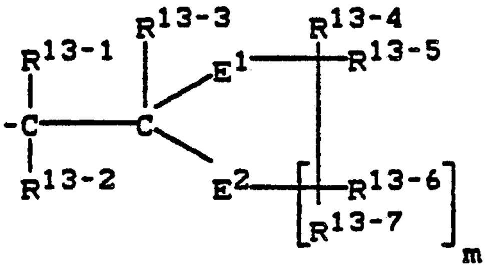

steht, wobei

$E^1$, $E^2$, $R^{13-1}$ bis $R^{13-7}$ und m die oben angegebene Bedeutung haben, und

G für Halogen, Alkylsulfonyloxy oder gegebenenfalls substituiertes Phenylsulfonyloxy steht,

in Gegenwart eines Verdünnungsmittels und gegebenenfalls in Gegenwart eines Säurebindemittels umsetzt.

Schließlich wurde gefunden, daß die neuen Aryloxy (bzw. thio)aminopyrimidine der Formel (I) sich durch hervorragende herbizide Wirkung auszeichnen.

Überraschenderweise besitzen die erfindungsgemäßen Aryloxy (bzw. thio)aminopyrimidine der Formel (I) wesentlich bessere herbizide Eigenschaften als die konstitutionell ähnlichsten vorbekannten Stoffe. So lassen sich die erfindungsgemäßen Stoffe der Formel (I) wesentlich besser zur Unkrautbekämpfung verwenden als z.B. 2-(3-Chlorphenoxy)-4-dimethylaminopyrimidin (bekannt aus EP-A 1 187/Beispiel 28), welches ein strukturell ähnlicher Wirkstoff ist.

Die erfindungsgemäßen Wirkstoffe sind im Gegensatz zu den vorbekannten Verbindungen durch einen 4-Aminosubstituenten im Pyrimidinring und durch die in der allgemeinen Formel (I) angegebenen unsubstituierten Positionen im Pyrimidinring und Aromaten ausgezeichnet.

Die Kohlenstoffketten in den einzelnen Resten sind jeweils geradkettig oder verzweigt. Die Substitution der verschiedenen Reste wie beispielsweise den aliphatischen, acyclischen oder aromatischen Resten kann jeweils einfach oder mehrfach, gleich oder verschieden sein.

Bevorzugt sind Aryloxy (bzw. thio)aminopyrimidine der Formel (I)

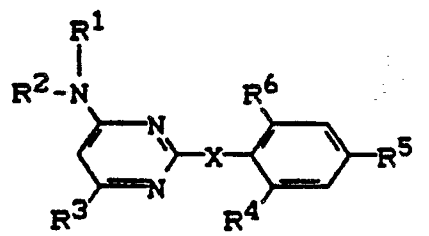

(I)

in welcher

$R^1$ für Wasserstoff, für Alkyl mit 1 bis 6 Kohlenstoffatomen, Alkoxyalkyl mit 1 bis 4 Kohlenstoffatomen in den einzelnen Alkylteilen, Cycloalkyl mit 3 bis 6 Kohlenstoffatomen oder Alkenyl mit 3 bis 6 Kohlenstoffatomen steht,

$R^2$ für Wasserstoff oder Alkyl mit 1 bis 6 Kohlenstoffatomen steht oder

$R^1$ und $R^2$ gemeinsam mit dem Stickstoffatom, an das sie gebunden sind, einen drei- bis sechsgliedrigen Ring, der gegebenenfalls 1 oder 2 weitere Heteroatome wie Sauerstoff und/oder Schwefel enthält, bilden können,

$R^3$ für Wasserstoff oder gegebenenfalls durch Halogen, $C_1$-$C_6$-Alkoxy und/oder $C_1$-$C_6$-Alkylthio substituiertes Alkyl mit 1 bis 6 Kohlenstoffatomen steht,

X für Sauerstoff oder Schwefel steht,

$R^4$ und $R^6$ unabhängig voneinander für Wasserstoff, Halogen, Nitro, gegebenenfalls durch Halogen substituiertes Alkyl mit 1 bis 6 Kohlenstoffatomen oder gegebenenfalls durch Halogen substituiertes Alkoxy mit 1 bis 6 Kohlenstoffatomen stehen und

$R^5$ für Wasserstoff, Halogen, Nitro, Hydroxy, gegebenenfalls durch Halogen, $C_1$-$C_6$-Alkoxy und/oder $C_1$-$C_6$-Alkylthio substituiertes Alkyl mit 1 bis 12 Kohlenstoffatomen, gegebenenfalls durch Halogen substituiertes Alkenyl mit 2 bis 12 Kohlenstoffatomen, gegebenenfalls durch Halogen substituiertes Alkinyl mit 2 bis 12 Kohlenstoffatomen, jeweils gegebenenfalls durch Halogen substituiertes Alkoxy, Alkoxyalkyloxy, Alkoxyalkoxyalkyloxy, Alkylthio, Alkylsulfoxy oder Alkylsulfonyl mit jeweils 1 bis 12 Kohlenstoffatomen in den einzelnen

Alkylteilen, jeweils gegebenenfalls durch Halogen, $C_1$-$C_6$-Alkoxy und/oder $C_1$-$C_6$-Alkylthio substituiertes Alkylcarbonyl oder Alkoxycarbonylalkyl mit jeweils 1 bis 10 Kohlenstoffatomen in den einzelnen Alkylteilen, für einen Rest der Formel

$-NR^7R^8$

steht, wobei
$R^7$ für Wasserstoff, gegebenenfalls durch Halogen substituiertes Alkyl mit 1 bis 6 Kohlenstoffatomen, Alkenyl oder Alkinyl mit jeweils 3 bis 6 Kohlenstoffatomen steht und
$R^8$ für Wasserstoff, Alkyl mit 1 bis 10 Kohlenstoffatomen, für einen Rest der Formel

- C -$R^9$
  ||
  B ·

steht, in welcher
B für Sauerstoff der Schwefel steht und
$R^9$ für Alkyl mit 1 bis 12 Kohlenstoffatomen, Alkenyl oder Alkinyl mit jeweils 2 bis 12 Kohlenstoffatomen steht, wobei Alkyl, Alkenyl und Alkinyl gegebenenfalls substituiert sind durch Halogen, Nitro, Cyano, gegebenenfalls durch Halogen und/oder Halogen-($C_1$-$C_4$)-alkylsubstituiertes Phenyl und/oder durch einen oder mehrere, insbesondere ein bis drei, gleiche oder verschiedene Reste der Formel

$-D-R^{10}$

in welcher
D für Sauerstoff, Schwefel, Sulfoxy oder Sulfonyl steht und
$R^{10}$ für Wasserstoff, jeweils gegebenenfalls durch Halogen und/oder $C_1$-$C_6$-Alkoxy substituiertes Alkyl mit 1 bis 6 Kohlenstoffatomen oder Alkenyl mit 2 bis 5 Kohlenstoffatomen, jeweils gegebenenfalls durch Halogen, $C_1$-$C_4$-Alkyl und/oder $C_1$-$C_4$-Alkoxy substituiertes Phenyl oder Naphthyl steht, oder
$R^9$ weiterhin für gegebenenfalls durch Halogen, $C_1$-$C_6$-Alkyl, $C_1$-$C_6$-Alkoxy und/oder Halogen-$C_1$-$C_6$-alkyl substituiertes Cycloalkyl mit 3 bis 8 Kohlenstoffatomen oder einen gegebenenfalls durch $C_1$-$C_6$-Alkyl und/oder $C_1$-$C_6$-Alkoxy substituierten Heterocyclus mit 3 bis 6 Ringgliedern und 1 oder 2 Sauerstoff- und/oder Schwefelatomen steht, oder
$R^5$ weiterhin für einen Rest der Formel

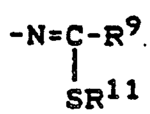

steht, in der
$R^9$ die oben angegebene Bedeutung hat und
$R^{11}$ für gegebenenfalls durch Halogen substituiertes Alkyl mit 1 bis 6 Kohlenstoffatomen, für Alkoxyalkyl mit 1 bis 6 Kohlenstoffatomen je Alkylteil, für Alkenyl oder Alkinyl mit jeweils 3 bis 6 Kohlenstoffatomen steht, oder
$R^5$ für einen Rest der Formel

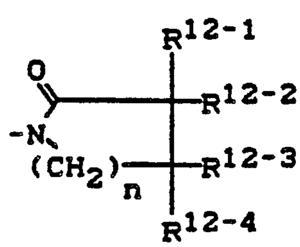

steht, wobei
n für die Zahl 1 oder 2 steht und
$R^{12-1}$, $R^{12-2}$, $R^{12-3}$ und $R^{12-4}$ unabhängig voneinander für Wasserstoff, Halogen oder Alkyl mit 1 bis 6

Kohlenstoffatomen stehen, oder
$R^5$ für einen Rest der Formel

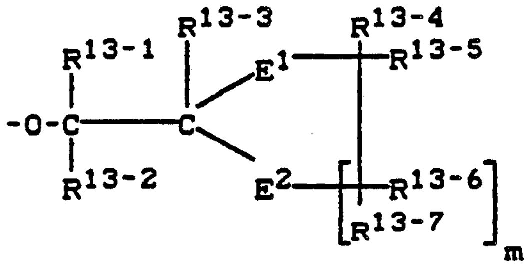

steht, in welcher
$E^1$ und $E^2$ unabhängig voneinander für Sauerstoff oder Schwefel stehen,
$R^{13-1}$ bis $R^{13-7}$ unabhängig voneinander für Wasserstoff oder Alkyl mit 1 bis 6 Kohlenstoffatomen stehen und
m für die Zahl 1 oder 2 steht.

Besonders bevorzugt sind Verbindungen der Formel (I), in welcher
$R^1$ für Wasserstoff, Alkyl oder Alkoxyalkyl mit jeweils 1 bis 4 Kohlenstoffatomen in den einzelnen Alkylteilen, Cycloalkyl mit 3 bis 6 Kohlenstoffatomen oder Alkenyl mit 3 bis 4 Kohlenstoffatomen steht,
$R^2$ für Wasserstoff oder Alkyl mit 1 bis 4 Kohlenstoffatomen steht oder
$R^1$ und $R^2$ gemeinsam mit dem Stickstoffatom, an das sie gebunden sind, für Aziridino, Pyrrolidino, Piperidino oder Morpholino stehen,
$R^3$ für Wasserstoff oder gegebenenfalls durch Halogen, $C_1$-$C_4$-Alkoxy und/oder $C_1$-$C_4$-Alkylthio substituiertes Alkyl mit 1 bis 4 Kohlenstoffatomen steht,
X für Sauerstoff oder Schwefel steht,
$R^4$ und $R^6$ unabhängig voneinander für Wasserstoff, Fluor, Chlor, Brom, Nitro, gegebenenfalls durch Fluor und/oder Chlor substituiertes Alkyl mit 1 bis 3 Kohlenstoffatomen oder gegebenenfalls durch Fluor und/oder Chlor substituiertes Alkoxy mit 1 bis 3 Kohlenstoffatomen stehen und
$R^5$ für Wasserstoff, Fluor, Chlor, Brom, Nitro, Hydroxy, gegebenenfalls durch Halogen, $C_1$-$C_4$-Alkoxy und/oder $C_1$-$C_4$-Alkylthio substituiertes Alkyl mit 1 bis 10 Kohlenstoffatomen, jeweils gegebenenfalls durch Halogen substituiertes Alkenyl oder Alkinyl mit jeweils 2 bis 9 Kohlenstoffatomen, jeweils gegebenenfalls durch Halogen substituiertes Alkoxy, Alkoxyalkyloxy, Alkoxyalkoxyalkyloxy, Alkylthio, Alkylsulfoxy oder Alkylsulfonyl mit jeweils 1 bis 10 Kohlenstoffatomen, jeweils gegebenenfalls durch Halogen, $C_1$-$C_4$-Alkoxy und/oder $C_1$-$C_4$-Alkylthio substituiertes Alkylcarbonyl oder Alkoxycarbonylalkyl mit jeweils 1 bis 8 Kohlenstoffatomen in den einzelnen Alkylteilen, für einen Rest der Formel

$-NR^7R^8$

steht, wobei
$R^7$ für Wasserstoff, Alkyl mit 1 bis 4 Kohlenstoffatomen, Alkenyl oder Alkinyl mit jeweils 3 bis 4 Kohlenstoffatomen steht und
$R^8$ für Wasserstoff, Alkyl mit 1 bis 8 Kohlenstoffatomen, einen Rest der Formel

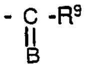

steht, in welcher
B für Sauerstoff oder Schwefel steht und
$R^9$ für Alkyl mit 1 bis 9 Kohlenstoffatomen, Alkenyl oder Alkinyl mit jeweils 2 bis 9 Kohlenstoffatomen steht, wobei Alkyl, Alkenyl und Alkinyl gegebenenfalls substituiert sind durch Halogen, Nitro, Cyano, gegebenenfalls durch Fluor, Chlor, Brom und/oder Halogen -($C_1$-$C_3$)-alkyl substituiertes Phenyl und/oder durch einen, zwei oder drei gleiche oder verschiedene Reste der Formel

$-D-R^{10}$

in welcher

D für Sauerstoff, Schwefel, Sulfoxy oder Sulfonyl steht und
$R^{10}$ für Wasserstoff, jeweils gegebenenfalls durch Halogen und/oder $C_1$-$C_4$-Alkoxy substituiertes Alkyl mit 1 bis 4 Kohlenstoffatomen oder Alkenyl mit 2 bis 4 Kohlenstoffatomen oder jeweils gegebenenfalls durch Halogen, $C_1$-$C_3$-Alkyl und/oder $C_1$-$C_3$-Alkoxy substituiertes Phenyl oder Naphthyl steht, oder
$R^9$ weiterhin für gegebenenfalls durch Halogen, $C_1$-$C_5$-Alkyl, $C_1$-$C_5$-Alkoxy und/oder Halogen-$C_1$-$C_3$-alkyl substituiertes Cycloalkyl mit 3 bis 6 Kohlenstoffatomen oder einen gegebenenfalls durch $C_1$-$C_4$-Alkyl und/oder $C_1$-$C_4$-Alkoxy substituierten Heterocyclus mit 3 bis 6 Ringgliedern und mit 1 oder 2 Sauerstoff- und/oder Schwefelatomen, insbesondere für den Heterocyclus

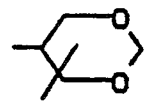 ,

steht und
$R^5$ weiterhin für einen Rest der Formel

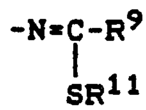

steht, in der
$R^9$ die oben angegebene Bedeutung hat und
$R^{11}$ für gegebenenfalls durch Fluor, Chlor und/oder Brom substituiertes Alkyl mit 1 bis 5 Kohlenstoffatomen, Alkoxyalkyl mit 1 bis 4 Kohlenstoffatomen je Alkylteil, für Alkenyl oder Alkinyl mit jeweils 3 bis 4 Kohlenstoffatomen steht, oder
$R^5$ für einen Rest der Formel

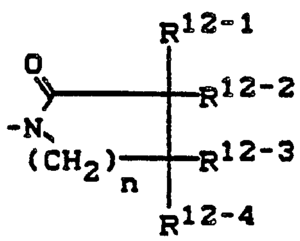

steht, wobei
n für die Zahl 1 oder 2 steht und
$R^{12-1}$, $R^{12-2}$, $R^{12-3}$ und $R^{12-4}$ unabhängig voneinander für Wasserstoff, Fluor, Chlor, Brom oder Alkyl mit 1 bis 4 Kohlenstoffatomen stehen, oder
$R^5$ für einen Rest der Formel

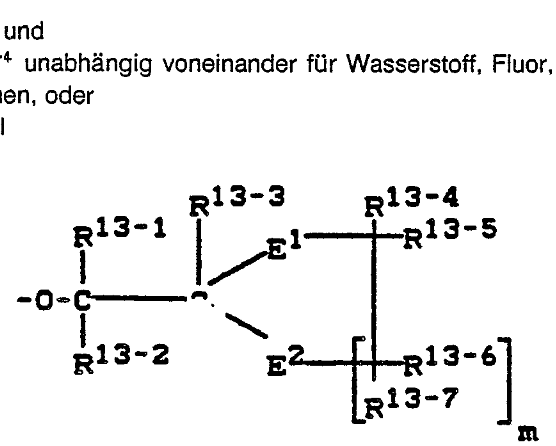

steht, in welcher
$E^1$ und $E^2$ unabhängig voneinander für Sauerstoff oder Schwefel stehen,
$R^{13-1}$ bis $R^{13-7}$ unabhängig voneinander für Wasserstoff oder Alkyl mit 1 bis 4 Kohlenstoffatomen stehen und

m für die Zahl 1 oder 2 steht.

Ganz besonders bevorzugt sind die Verbindungen der Formel (I), in welcher

$R^1$ für Wasserstoff, Methyl, Ethyl, n-Propyl, i-Propyl, t-Butyl, Methoxyethyl, Ethoxyethyl, Cyclopropyl oder Allyl steht und

$R^2$ für Wasserstoff, Methyl, Ethyl, n-Propyl oder i-Propyl steht,

$R^1$ und $R^2$ gemeinsam mit dem Stickstoffatom, an das sie gebunden sind, für Aziridino, Pyrrolidino, Piperidino oder Morpholino steht,

$R^3$ für Wasserstoff oder gegebenenfalls durch Fluor, Chlor, Methoxy, Ethoxy, n-Propoxy, i-Propoxy, Methylthio und/oder Ethylthio substituiertes Alkyl mit 1 bis 3 Kohlenstoffatomen steht,

X für Sauerstoff oder Schwefel steht,

$R^4$ und $R^6$ unabhängig voneinander für Wasserstoff, Fluor, Chlor, Nitro, jeweils gegebenenfalls durch Fluor und/oder Chlor substituiertes Methyl oder Ethyl, für jeweils gegebenenfalls durch Fluor und/oder Chlor substituiertes Methoxy oder Ethoxy stehen und

$R^5$ für Wasserstoff, Fluor, Chlor, Nitro, Hydroxy, gegebenenfalls durch Fluor, Chlor, Methoxy, Ethoxy, n-Propoxy, i-Propoxy, Methylthio und/oder Ethylthio substituiertes Alkyl mit 1 bis 9 Kohlenstoffatomen, jeweils gegebenenfalls durch Fluor und/oder Chlor substituiertes Allyl oder Propargyl, jeweils gegebenenfalls durch Fluor und/oder Chlor substituiertes Alkoxy, Alkoxyalkyloxy, Alkoxyalkoxyalkyloxy, Alkylthio, Alkylsulfoxy oder Alkylsulfonyl mit jeweils 1 bis 9 Kohlenstoffatomen, jeweils gegebenenfalls durch Fluor, Chlor, Methoxy, Ethoxy, Methylthio und/oder Ethylthio substituiertes Alkylcarbonyl oder Alkoxycarbonyl mit jeweils 1 bis 6 Kohlenstoffatomen in den eizelnen Alkylteilen, für einen Rest der Formel

$$-NR^7R^8$$

steht, wobei

$R^7$ für Wasserstoff, Methyl, Ethyl, Allyl oder Propargyl steht und

$R^8$ für Wasserstoff, Alkyl mit 1 bis 6 Kohlenstoffatomen, einen Rest der Formel

$$-\underset{\underset{B}{\parallel}}{C}-R^9$$

steht, in welcher

B für Sauerstoff oder Schwefel steht und

$R^9$ für jeweils gegebenenfalls durch Fluor, Chlor, Brom, Iod, Cyano, gegebenenfalls durch Fluor, Chlor und/oder Halogen $-(C_1-C_2)$-alkyl, wobei Halogen insbesondere für Fluor und/oder Chlor steht, substituiertes Phenyl und/oder durch einen, zwei oder drei gleiche oder verschiedene Reste der Formel

$$-D-R^{10}$$

substituiertes Alkyl mit 1 bis 6 Kohlenstoffatomen, Alkenyl mit 2 bis 6 Kohlenstoffatomen oder Alkinyl mit 2 bis 6 Kohlenstoffatomen steht, wobei

D für Sauerstoff, Schwefel, Sulfoxy oder Sulfonyl steht und

$R^{10}$ für Wasserstoff, jeweils gegebenenfalls durch Fluor, Chlor, Brom und/oder $C_1$-$C_4$-Alkoxy substituiertes Alkyl mit 1 bis 4 Kohlenstoffatomen oder Alkenyl mit 2 bis 4 Kohlenstoffatomen oder gegebenenfalls durch Fluor, Chlor, $C_1$-$C_3$-Alkyl und/oder $C_1$-$C_3$-Alkoxy substituiertes Phenyl steht, oder

$R^9$ weiterhin für einen Cycloalkylrest mit 3 bis 6 Kohlenstoffatomen steht, der gegebenenfalls durch Fluor, Chlor, $C_1$-$C_3$-Alkyl, $C_1$-$C_3$-Alkoxy und/oder Trifluormethyl substituiert ist, oder für einen gegebenenfalls durch $C_1$-$C_4$-Alkyl oder $C_1$-$C_4$-Alkoxy substituierten gesättigten 1 oder 2 Sauerstoff- oder Schwefelatome enthaltenden heterocyclischen Ring mit 3 bis 6 Ringgliedern, insbesondere für den Heterocyclus

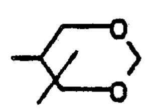 ,

steht, und

$R^5$ weiterhin für einen Rest der Formel

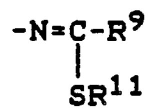

steht, in der
$R^9$ die oben angegebene Bedeutung hat und
$R^{11}$ für Methyl, Ethyl oder Isopropyl, für Methoxymethyl, Methoxyethyl, Ethoxymethyl, Ethoxyethyl, Allyl oder Propargyl steht, oder
$R^5$ weiterhin für einen Rest der Formel

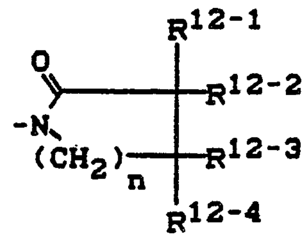

steht, wobei
n für die Zahl 1 oder 2 steht und
$R^{12-1}$ bis $R^{12-4}$ unabhängig voneinander für Wasserstoff, Fluor, Chlor, Methyl, Ethyl, n-Propyl oder i-Propyl stehen, oder
$R^5$ weiterhin für einen Rest der Formel

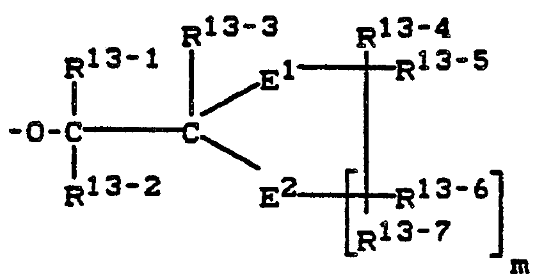

steht, in welcher
$E^1$ und $E^2$ unabhängig voneinander für Sauerstoff oder Schwefel stehen,
$R^{13-1}$ bis $R^{13-7}$ unabhängig voneinander für Wasserstoff, Methyl oder Ethyl stehen und
m für die Zahl 1 oder 2 steht.

Ganz besonders hervorzuheben ist die Gruppe von Verbindungen der Formel (I), in welcher X für Schwefel steht und die restlichen Substituenten die oben als ganz besonders bevorzugt angegebene Bedeutung haben.

Verwendet man gemäß Verfahren (A) 4-Amino-6-methyl-2-methylsulfonylpyrimidin und p-Methylthiophenol als Ausgangsstoffe, so kann der Verlauf des erfindungsgemäßen Verfahrens (A) durch das folgende Formelschema wiedergegeben werden:

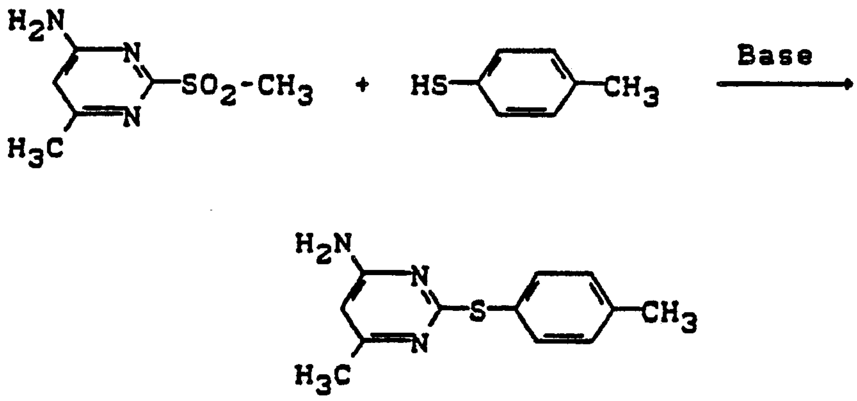


Verwendet man gemäß Verfahren (B) 4-Chlor-6-methyl-2-(4-nitrophenylthio)pyrimidin und Ammoniak als Ausgangsstoffe, so kann der Verlauf des erfindungsgemäßen Verfahrens durch das folgende Formelschema wiedergegeben werden:

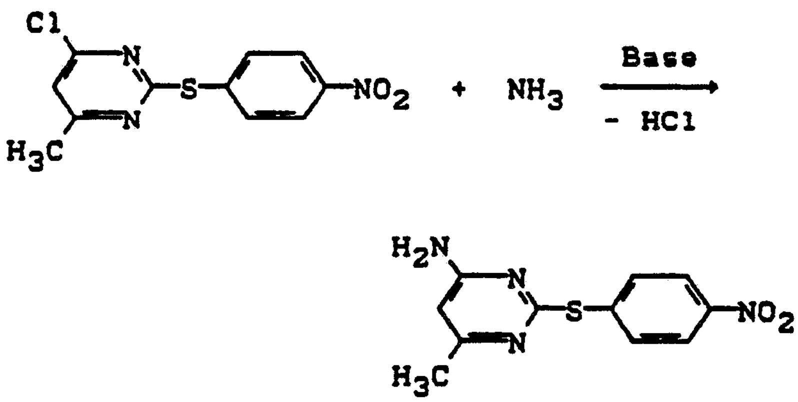


Verwendet man gemäß Verfahren ($C-C_1$) 4-Amino-6-methyl-2-(4-nitrophenylthio)pyrimidin und Wasserstoff als Ausgangsmaterialien so kann der Verlauf des erfindungsgemäßen Verfahrens durch das folgende Formelschema wiedergegeben werden:

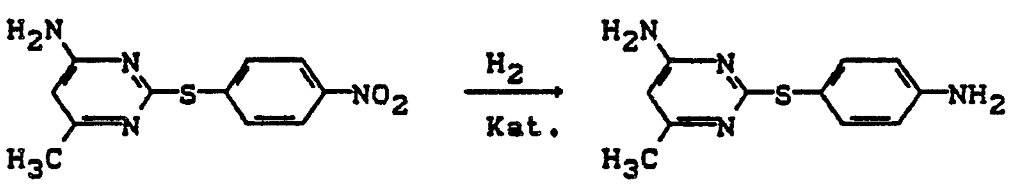


Verwendet man gemäß Verfahren ($C-C_2$/Variante α) 4-Amino-6-methyl-2-(4-aminophenylthio)pyrimidin und Acetylchlorid als Ausgangsstoffe, so wird der Verlauf des erfindungsgemäßen Verfahrens durch folgendes Formelschema wiedergegeben:

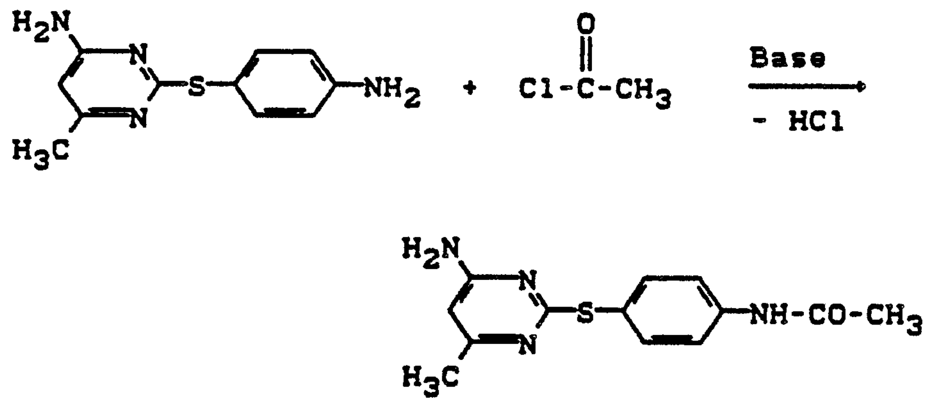


Verwendet man gemäß Verfahren (C-$C_2$/Variante β) 4-Amino-6-methyl-2-(4-aminophenylthio)pyrimidin und Isobuttersäureanhydrid als Ausgangsstoffe, so kann der Verlauf des erfindungsgemäßen Verfahrens durch folgendes Formelschema wiedergegeben werden:

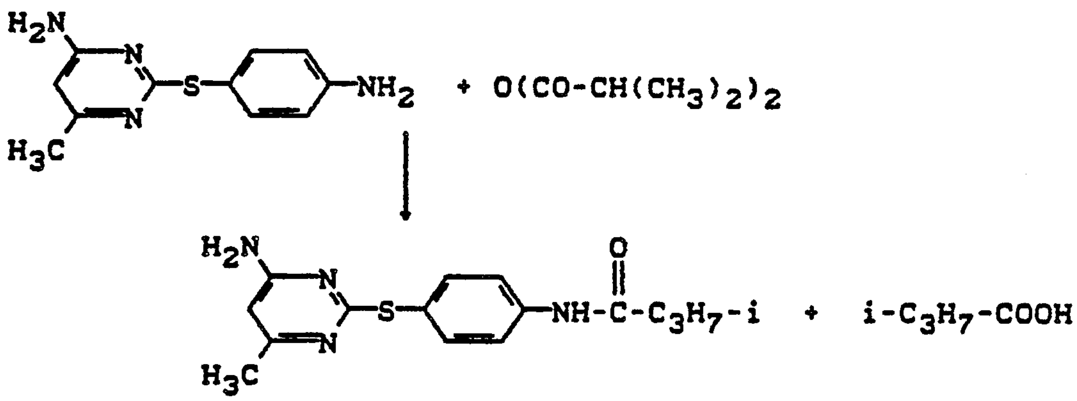

Verwendet man gemäß Verfahrens (C-$C_2$/Variante γ) 4-Amino-6-methyl-2-(4-aminophenylthio)pyrimidin und Pivaloylkohlensäureethylesteranhydrid als Ausgangsstoffe, so kann der Verlauf des erfindungsgemäßen Verfahrens durch folgendes Formelschema wiedergegeben werden:

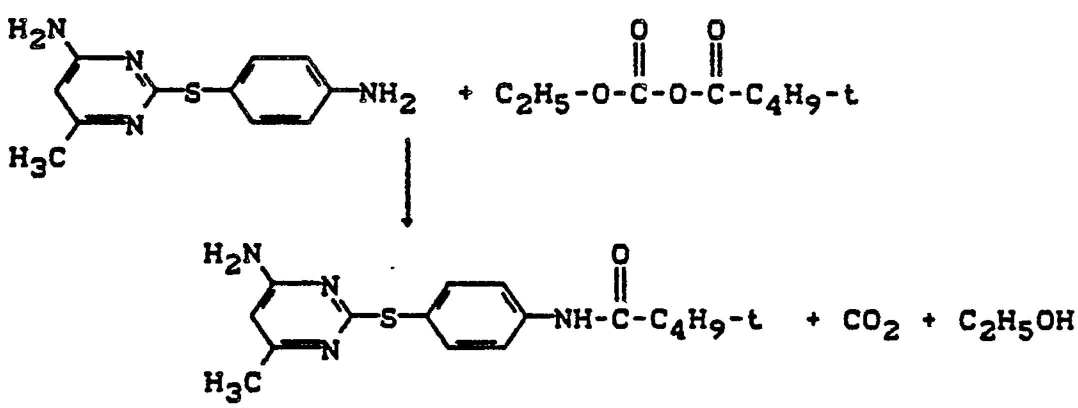

Verwendet man gemäß Verfahren (C-$C_2$/Variante δ) 4-Amino-6-methyl-2-(4-aminophenylthio)pyrimidin und 4-Chlor-2,2-dimethyl-butansäurechlorid als Ausgangsstoffe, so kann der Verlauf des erfindungsgemäßen Verfahrens durch folgendes Formelschema wiedergegeben werden:

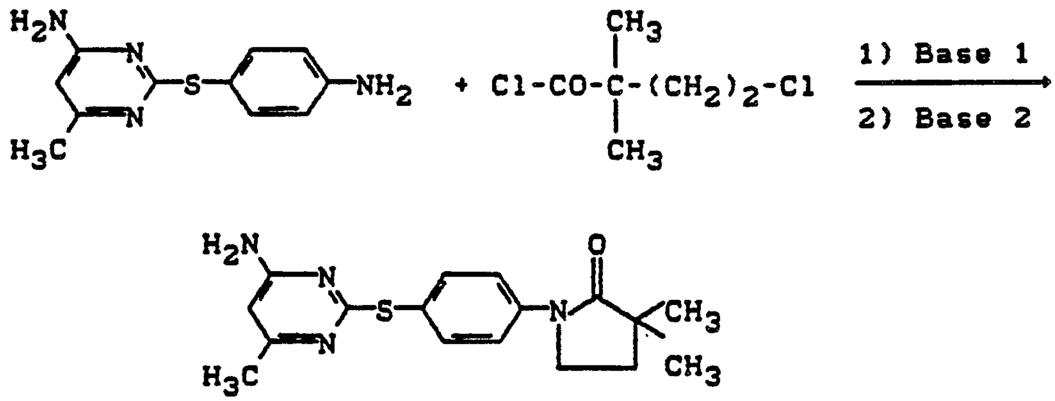


Verwendet man gemäß Verfahren (D) 4-Dimethylamino-6-methyl-2-(4-t-butylcarbonylaminophenylthio)-pyrimidin und Lawesson-Reagenz als Ausgangsstoffe, so wird der Verlauf des erfindungsgemäßen Verfahrens durch folgendes Schema beschrieben:

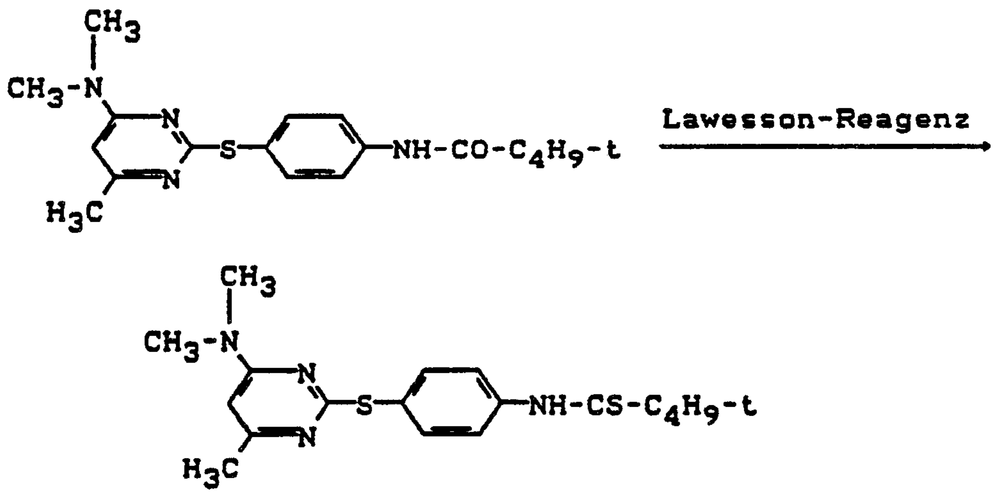


Verwendet man gemäß Verfahren (E) 4-Dimethylamino-6-methyl-2-(4-t-butylthiocarbonylaminophenylthio)pyrimidin und Methyliodid als Ausgangstoffe, so wird der Verlauf des erfindungsgemäßen Verfahrens durch folgendes Schema beschrieben:

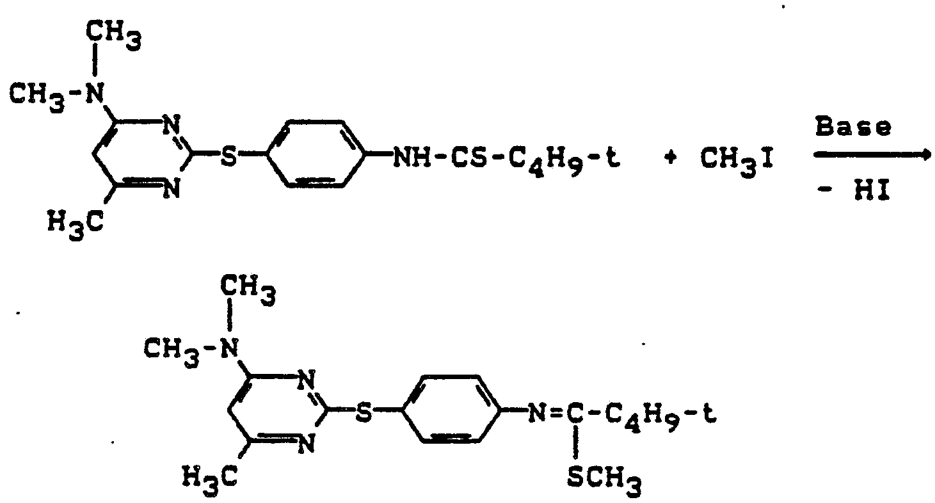


Verwendet man gemäß Verfahren (F) 4-Dimethylamino-6-methyl-2-(4-hydroxyphenylthio)pyrimidin und

2-Methyl-2-methoxy-1-propyl-tosylat als Ausgangsstoffe, so kann der Verlauf des erfindungsgemäßen Verfahrens durch folgendes Formelschema wiedergegeben werden:

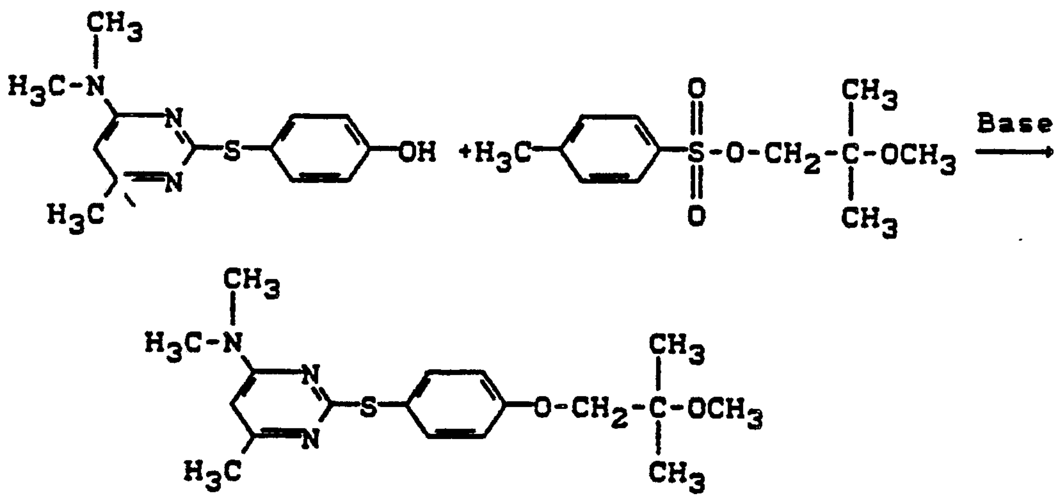


Die bei dem erfindungsgemäßen Verfahren (A) eingesetzten Pyrimidinderivate der Formel (II) sind bekannt oder können nach bekannten Verfahren der organischen Chemie erhalten werden (vgl. A. Weissberger: The Chemistry of heterocyclic compounds; The Pyrimidines, 1962 Interscience, New York).

In dieser Formel (II) stehen die Reste $R^1$, $R^2$, $R^3$ vorzugsweise für diejenigen Reste, die bereits im Zusammenhang mit der Beschreibung der erfindungsgemäßen Stoffe der allgemeinen Formel (I) vorzugsweise erwähnt wurden. Der Rest $R^{14}$ steht bevorzugt für Chlor, Fluor und Methylsulfonyl.

Die ebenfalls bei diesem Verfahren eingesetzten Phenole bzw. Thiophenole werden durch die allgemeine Formel (III) beschrieben. In ihr stehen die Reste $R^4$, $R^5$, $R^6$ und X für die Reste, die schon bei der Beschreibung von Verbindungen der Formel (I) als bevorzugt bzw. besonders bevorzugt erwähnt wurden. Die Verbindungen der Formel (III) sind teilweise bekannt (vgl. DE-OS 2 156 345). Teilweise sind sie neu und bereits Gegenstand einer parallel eingereichten Anmeldung und Gegenstand dieser Anmeldung.

Neu und Gegenstand dieser Anmeldung sind die Thiophenole der Formel (IIIa)

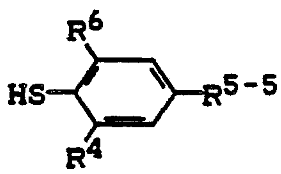

(IIIa)

in welcher

$R^4$ und $R^6$ unabhängig voneinander für Wasserstoff, Halogen, Nitro oder für jeweils gegebenenfalls substituiertes Alkyl oder Alkoxy stehen und

$R^{5-5}$ für gegebenenfalls substituiertes Alkyl mit mindestens einem tertiären Kohlenstoffatom steht, mit der Maßgabe, daß $R^{5-5}$ nicht für tert.-Butyl steht, wenn $R^4$ und $R^6$ gleichzeitig für Wasserstoff stehen.

Bevorzugt sind die Verbindungen der Formel (IIIa), in welcher

$R^4$ und $R^6$ unabhängig voneinander für Wasserstoff, Halogen, gegebenenfalls durch Halogen substituiertes Alkyl mit 1 bis 6 Kohlenstoffatomen oder gegebenenfalls durch Halogen substituiertes Alkoxy mit 1 bis 6 Kohlenstoffatomen stehen und

$R^{5-5}$ für Alkyl mit 5 bis 12 Kohlenstoffatomen mit mindestens einem tertiären Kohlenstoffatom steht, welches durch Halogen, $C_1$-$C_6$-Alkoxy und/oder $C_1$-$C_6$-Alkylthio substituiert sein kann.

Besonders bevorzugt sind die Verbindungen der Formel (IIIa), in welcher

$R^4$ und $R^6$ unabhängig voneinander für Wasserstoff, Fluor, Chlor, gegebenenfalls durch Fluor und/oder Chlor substituiertes Alkyl mit 1 bis 3 Kohlenstoffatomen oder gegebenenfalls durch Fluor und/oder Chlor substituiertes Alkoxy mit 1 bis 3 Kohlenstoffatomen stehen und

$R^{5-5}$ für jeweils gegebenenfalls durch Halogen, $C_1$-$C_4$-Alkoxy und/oder $C_1$-$C_4$-Alkylthio substituiertes tert.-Butylethyl oder tert.-Butylpropyl steht.

Insbesondere steht $R^{5-5}$ in Formel (IIIa) für $-CH_2-CH_2-C(CH_3)_3$ oder $-CH_2-CH_2-CH_2-C(CH_3)_3$.

Insbesondere sind die Verbindungen

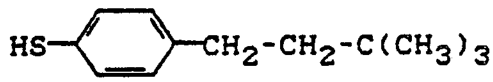

und

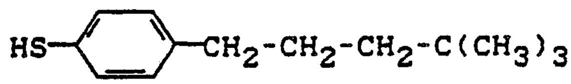

genannt.

Die Verbindungen der Formel (III) lassen sich nach prinzipiell bekannten Verfahren herstellen.

So erhält man beispielsweise (Thio)phenole der Formel (III), in denen $R^5$ für eine elektronenanziehende Gruppe steht, wenn man Halogenbenzole der Formel (XIIIa)

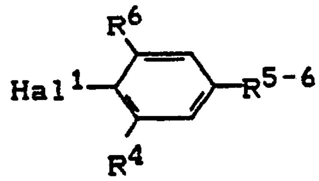

(XIIIa)

in welcher
$R^4$ und $R^6$ die oben angegebene Bedeutung haben und
$R^{5-6}$ für Nitro, Vinyl, gegebenenfalls substituiertes Alkylcarbonyl, Alkylsulfoxy oder Alkylsulfonyl steht und
$Hal^1$ für Halogen, insbesondere für Fluor, Chlor oder Brom, steht,
mit Alkalimetalldisulfiden, insbesondere Natriumdisulfid, nach den in DE-OS 2 156 345 beschriebenen Verfahrensbedingungen umsetzt.

Die neuen Thiophenole der Formel (IIIa) erhält man beispielsweise, wenn man Verbindungen der Formel (IIIb)

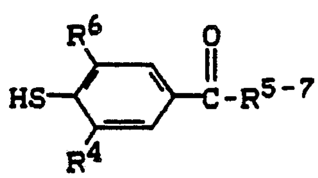

(IIIb)

in welcher
$R^4$ und $R^6$ die oben angegebene Bedeutung haben und
$R^{5-7}$ für gegebenenfalls substituiertes Alkyl mit mindestens einem tertiären Kohlenstoffatom steht,
mit Reduktionsmitteln wie beispielsweise Natriumborhydrid in Gegenwart eines Verdünnungsmittels wie beispielsweise Diethylenglykoldimethylether bei Temperaturen zwischen 20 °C und 140 °C umsetzt (vgl. Herstellungsbeispiele).

Die Verbindungen der Formel (IIIb) fallen unter die Formel (III) und sind dort beschrieben. $R^{5-7}$ in Formel (IIIb) steht vorzugsweise für Alkyl mit 4 bis 11 Kohlenstoffatomen mit mindestens einem tertiären Kohlenstoffatom, welches durch Halogen, $C_1$-$C_6$-Alkoxy und/oder $C_1$-$C_6$-Alkylthio substituiert sein kann. Besonders bevorzugt steht $R^{5-7}$ für tert.-Butylmethyl oder tert.-Butylethyl, welche durch Halogen, $C_1$-$C_4$-Alkoxy und/oder $C_1$-$C_4$-Alkylthio substituiert sein können.

Weiterhin erhält man Verbindungen der Formel (IIIb), wenn man Halogenbenzole der Formel (XIIIb)

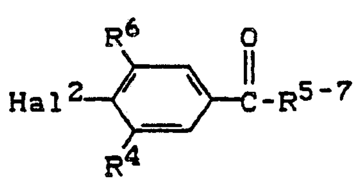

(XIIIb)

in welcher
$R^4$, $R^{5-7}$ und $R^6$ die oben angegebene Bedeutung haben und
$Hal^2$ für Halogen, insbesondere Fluor, Chlor oder Brom steht,
mit Natriumhydrogensulfid in Gegenwart eines Verdünnungsmittels wie beispielsweise N-Methylpyrrolidon bei Temperaturen zwischen 50 °C und 200 °C umsetzt (vgl. Herstellungsbeispiele).

Die Halogenbenzole der Formel (XIIIa) und (XIIIb) fallen unter die allgemeine Formel (XIII) und sind dort näher beschrieben. In der Formel (XIIIa) steht $R^{5-6}$ vorzugsweise für Nitro, gegebenenfalls durch Halogen, $C_1$-$C_6$-Alkoxy und/oder $C_1$-$C_6$-Alkylthio substituiertes Alkylcarbonyl mit 1 bis 10 Kohlenstoffatomen, Alkylsulfoxy oder Alkylsulfonyl mit jeweils 1 bis 12 Kohlenstoffatomen. In der Formel (XIIIB) hat $R^{5-7}$ vorzugsweise bzw. besonders bevorzugt die oben bei der Beschreibung der Stoffe der Formel (IIIa) für diesen Substituenten bevorzugt bzw. besonders bevorzugt angegebene Bedeutung.

Als Säurebindemittel können bei der Durchführung des Verfahrens (A) alle üblicherweise für derartige Umsetzungen verwendbaren Säureakzeptoren verwendet werden. Vorzugsweise verwendbar sind Erdalkalioxide, Alkali- und Erdalkalihydroxide und -carbonate, wie Natriumhydroxid, Kaliumhydroxid, Calciumoxid, Natriumcarbonat und Kaliumcarbonat, ferner Alkali-alkoholate, -amide und -hydride, wie z.B. Natriummethylat, Natriumethylat, Kalium-tert.-butylat, Natriumamid und Natriumhydrid.

Als Verdünnungsmittel können bei der Durchführung des Verfahrens (A) alle üblichen inerten organischen Solventien verwendet werden. Vorzugsweise infrage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, N-Methylpyrrolidon, Sulfolan und Dimethylformamid.

Die Reaktionstemperaturen können bei der Durchführung des Verfahrens (A) innerhalb eines größeren Bereiches variiert werden. Im allgemeinen arbeitet man bei Temperaturen zwischen 0 °C und 200 °C, vorzugsweise zwischen 50 °C und 150 °C.

Die Umsetzung nach dem Verfahren (A) wird im allgemeinen unter Normaldruck vorgenommen.

Bei der Durchführung des Verfahrens (A) setzt man die Ausgangsstoffe der Formeln (II) und (III) im allgmeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem erfindungsgemäßen Verfahren (B) eingesetzten Halogenpyrimidinderivate sind teilweise bekannt oder können nach bekannten Verfahren erhalten werden. Sie sind durch die Formel (IV) allgemein definiert. In der Formel (IV) stehen die Reste $R^3$, $R^4$, $R^5$ und $R^6$ bevorzugt oder besonders bevorzugt für die Bedeutungen, die schon bei der Beschreibung der erfindungsgemäßen Stoffe der allgemeinen Formel (I) für diese Reste erwähnt wurde, mit der Maßgabe, daß mindestens einer der Reste $R^4$, $R^5$ oder $R^6$ starke Elektronenakzeptoreigenschaften besitzt wie z.B. Nitro oder Halogenalkyl. Hal steht vorzugsweise für Fluor, Chlor oder Brom.

Die Verbindungen der allgemeinen Formel (IV) werden erhalten, indem man in einem ersten Reaktionsschritt (Stufe 1) Thiopyrimidinderivate der allgemeinen Formel (XII)

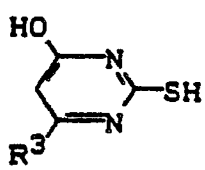

(XII)

in welcher
$R^3$ die oben angegebene Bedeutung hat,
mit Halogenbenzolen der allgemeinen Formel (XIII)

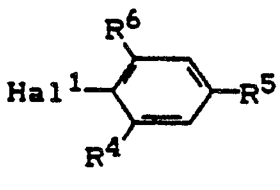

(XIII)

in welcher

$R^4$, $R^5$ und $R^6$ die oben angegebene Bedeutung haben und
$Hal^1$ für Halogen, insbesondere für Fluor, Chlor oder Brom steht,
gegebenenfalls in Gegenwart eines Verdünnungsmittels und gegebenenfalls in Gegenwart eines Säurebindemittels umsetzt,
zu Verbindungen der allgemeinen Formel (XIV)

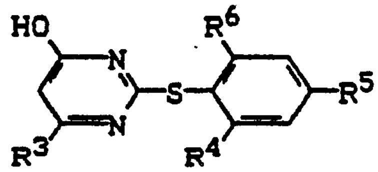

(XIV)

in welcher
$R^3$, $R^4$, $R^5$ und $R^6$ die oben angegebene Bedeutung haben.

Die Verbindungen der allgemeinen Formel (XIV) werden in einem zweiten Reaktionsschritt mit Halogenierungsmitteln wie Phosphoroxychlorid, Phosphor-V-chlorid, Thionylchlorid oder Phosgen in die Verbindungen der allgemeinen Formel (IV)

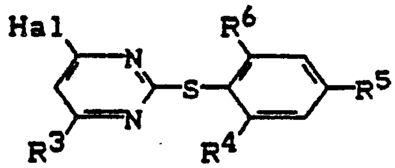

(IV)

in der $R^3$, $R^4$, $R^5$, $R^6$ und Hal die oben angegebene Bedeutung haben, übergeführt.

Die Thiopyrimidine der allgemeinen Formel (XII) sind bekannte Verbindungen (s. hierzu A. Weissberger, The Pyrimidines Bd. I, S. 272, Wiley Interscience (1962)).

In Formel (XII) steht der Rest $R^3$ bevorzugt bzw. besonders bevorzugt für die Bedeutung, die schon bei der Beschreibung der erfindungsgemäßen Stoffe (I) als bevorzugt bzw. besonders bevorzugt für diesen Rest erwähnt wurde.

Die Halogenbenzole der allgemeinen Formel (XIII) in der $R^4$, $R^5$ und $R^6$ dieselbe Bedeutung haben, die schon bei der Beschreibung der Verbindungen der allgemeinen Formel (IV) angegeben wurde und die auch bei der Beschreibung der erfindungsgemäßen Stoffe der allgemeinen Formel (I) als bevorzugt bzw. besonders bevorzugt definiert wurden, sind bekannt oder können nach bekannten Verfahren erhalten werden.

Die bei der zweiten Verfahrensstufe eingesetzten Halogenierungsmittel sind bekannte Verbindungen. Beispielhaft seien Phosphoroxychlorid, Thionylchlorid, Phosphor-V-chlorid oder Phosgen erwähnt (vgl. hierzu: A. Weissberger, The Pyrimidines Bd. I, S. 162-168, Wiley Interscience (1962)).

Die bei der Durchführung des erfindungsgemäßen Verfahrens (1. Stufe) zur Herstellung der Verbindungen der Formel (IV) eingesetzten Pyrimidinderivate der Formel (XII) und die Halogenbenzolderivate der allgemeinen Formel (XIII) werden im allgemeinen angenähert in äquivalenten Mengen eingesetzt. Es ist jedoch auch möglich, die eine oder andere Komponente in einem größeren Überschuß einzusetzen.

Als Säurebindemittel werden starke Basen wie Alkalimetall- oder Erdalkalimetallhydroxide oder -alkoholate verwendet. Das Säurebindemittel wird mindestens in doppelt äquivalenter Menge, bezogen auf das eingesetzte Thiopyrimidin (XII), eingesetzt.

Als Verdünnungsmittel eignen sich inbesondere starke polare Solventien wie Dimethylsulfoxid, Sulfolan, N-Methylpyrrolidon oder Dimethylformamid.

Die Reaktion wird bei Temperaturen zwischen 50 und 200 °C, vorzugsweise zwischen 75 und 150 °C, durchgeführt.

Die bei der Durchführung des zweiten Reaktionsschrittes eingesetzten Pyrimidinderivate der allgemeinen Formel (XIV) und das Hologenierungsmittel werden im allgemeinen in äquivalenten Mengen eingesetzt. Vorzugsweise wird das Halogenierungsmittel im Überschuß eingesetzt.

Die 2. Stufe des Verfahrens kann in An- oder Abwesenheit eines Verdünnungsmittels durchgeführt werden. Als Verdünnungsmittel kann auch das eingesetzte Halogenierungsmittel wie Thionylchlorid oder Phosphoroxychlorid in einem geeigneten Überschuß verwendet werden. Ebenso kommen als Verdünnungsmittel alle gegen halogenierende Agentien inerten Solventien in Betracht. Vorzugsweise sind verwendbar

Kohlenwasserstoffe wie Benzin, Benzol, Toluol, Xylol, Tetralin, Halogenkohlenwasserstoffe wie Methylenchlorid, Chloroform, Tetrachlorkohlenstoff, Chlorbenzol, o-Dichlorbenzol, darüber hinaus Ether wie Diethylether, Tetrahydrofuran und Dioxan.

Der zweite Reaktionsschritt zur Herstellung von Verbindungen der Formel (IV) wird bei Temperaturen zwischen 0 und 150 °C, vorzugsweise zwischen 50 und 125 °C, durchgeführt.

Als Katalysatoren werden bevorzugt, N,N-Dimethylanilin und Dimethylformamid eingesetzt.

Die bei dem erfindungsgemäßen Verfahren (B) benötigten Amine der allgemeinen Formel (V) sind bekannte Verbindungen der organischen Chemie. In der Formel (V) haben die Reste $R^1$ und $R^2$ bevorzugt bzw. besonders bevorzugt die Bedeutung, die schon bei der Beschreibung der Stoffe der allgemeinen Formel (I) bevorzugt bzw. besonders bevorzugt genannt wurden.

Als Verdünnungsmittel können bei dem erfindungsgemäßen Verfahren (B) alle üblichen organischen Solventien und Wasser eingesetzt werden.

Vorzugsweise verwendet man Kohlenwasserstoffe wie Benzin, Benzol, Toluol, Xylol, Tetralin, ferner Halogenkohlenwasserstoffe wie Methylenchlorid, Chloroform, Tetrachlorkohlenstoff, Chlorbenzol, o-Dichlorbenzol, außerdem Ketone wie Aceton und Methylisopropylketon, weiterhin Ether wie Diethylether, Tetrahydrofuran und Dioxan, darüber hinaus Carbonsäureester wie Ethylacetat und auch stark polare Solventien wie Dimethylsulfoxid oder Sulfolan.

Die Reaktionstemperaturen können in einem breiten Bereich variiert werden. Man setzt die Stoffe im allgemeinen bei Temperaturen zwischen 0 und 300 °C, vorzugsweise zwischen 50 und 150 °C, um.

Der Druck kann beim erfindungsgemäßen Verfahren (B) in breiten Bereichen variiert werden. Im allgemeinen arbeitet man bei einem Druck von 1 bis 100 atm., bevorzugt bei einem Druck von 1 bis 20 atm.

Als Säurebindemittel kommen bei der Umsetzung nach dem erfindungsgemäßen Verfahren (B) alle üblichen Säureakzeptoren in Betracht.

Vorzugsweise verwendbar sind tertiäre Amine wie Triethylamin, Pyridin und N,N-Dimethylanilin, ferner Erdalkalimetalloxide wie Calciumoxid, Carbonate wie Natriumcarbonat, Kaliumcarbonat und Calciumcarbonat. Ebenfalls ist es möglich, die jeweiligen Verbindungen der Formel (V) als Säurebindemittel zu verwenden. Die betreffende Verbindung muß dann zumindest in solcher Menge eingesetzt werden, daß der freiwerdende Halogenwasserstoff gebunden wird.

Bei der Durchführung des erfindungsgemäßen Verfahrens (B) werden die Ausgangsstoffe der allgemeinen Formeln (IV) und (V) im allgemeinen in äquivalenten Mengen verwendet, vorzugsweise setzt man pro Mol Halogenpyrimidinderivat der Formel (IV) 1,2 bis 20 Mol an Aminkomponente der Formel (V) ein.

Die Aufarbeitung erfolgt nach üblichen Methoden.

Als Reduktionsmittel kommen bei dem Verfahren ($C$-$C_1$) alle diejenigen Stoffe infrage, die üblicherweise zur Reduktion aromatischer Nitroverbindungen eingesetzt werden. Vorzugsweise verwendbar sind elementare Metalle, wie Eisen, Zink und Zinn, ferner Metallverbindungen in niederen Wertigkeitsstufen, wie Eisen-(II)- und Zinn(II)-Salze, und außerdem Nichtmetall-Verbindungen in niederen Wertigkeitsstufen, wie z.B. Salze des Schwefelwasserstoffes, Alkalisulfite und Alkalidithionite. Im übrigen kann die Reduktion auch durch katalytische Hydrierung mit Wasserstoff in Gegenwart eines Katalysators, wie z.B. Raney-Nickel, erfolgen.

Als Verdünnungsmittel kommen bei dem Verfahren ($C$-$C_1$) alle üblichen für derartige Reduktionen geeigneten organischen Solventien in Betracht.

Die Reaktionstemperaturen können bei Verfahren $C$-$C_1$) innerhalb eines größeren Bereiches variiert werden. Sie entsprechen den Temperaturen, die bei analogen Reaktionen angewandt werden. Im allgemeinen arbeitet man bei Temperaturen zwischen 0 und 150 °C, vorzugsweise zwischen 10 und 100 °C.

Die Durchführung der Reduktion nach dem Verfahren ($C$-$C_1$) und die Aufarbeitung des anfallenden Reaktionsgemisches erfolgen nach üblichen Methoden.

Die zur Durchführung des erfindungsgemäßen Verfahrens ($C$-$C_2$) benötigten Aryloxy (bzw. thio)-aminopyrimidine der Formel (Ib) sind erfindungsgemäße Verbindungen und erhältlich nach Verfahren ($C$-$C_1$).

In dieser Formel stehen die Reste $R^1$, $R^2$, $R^3$, $R^4$, $R^6$ und X vorzugsweise bzw. besonders bevorzugt für die Bedeutungen, die bereits bei der Beschreibung der erfindungsgemäßen Stoffe der Formel (I) bevorzugt bzw. besonders bevorzugt für diese Reste genannt wurden.

Die als Reaktionskomponenten weiterhin benötigten Säurehalogenide (Verfahren $C$-$C_2$/Variante $\alpha$) sind durch die Formel (VI) eindeutig definiert. In dieser Formel hat $R^9$ vorzugsweise diejenige Bedeutung, die bereits im Zusammenhang mit der Beschreibung der erfindungsgemäßen Stoffe der Formel (I) vorzugsweise für diesen Rest genannt wurde. Hal steht vorzugsweise für Fluor, Chlor oder Brom.

Die Säurehalogenide der Formel (VI) sind bekannt oder lassen sich nach im Prinzip bekannten Methoden in einfacher Weise herstellen.

Als Säurebindemittel kommen bei der Umsetzung nach dem erfindungsgemäßen Verfahren (C-$C_2$/Variante α) alle üblichen Säureakzeptoren in Betracht. Vorzugsweise verwendbar sind tertiäre Amine, wie Triethylamin, Pyridin und N,N-Dimethyl-anilin, ferner Erdalkalimetalloxide, wie Calciumoxid, Alkali- und Erdalkalicarbonate, wie Natriumcarbonat, Kaliumcarbonat und Calciumcarbonat. Es ist auch möglich, die jeweiligen Verbindungen der Formel (Ib) gleichzeitig als Säurebindemittel zu verwenden. Dazu muß die betreffende Verbindung dann zumindest in solcher Menge eingesetzt werden, daß der freiwerdende Halogenwasserstoff gebunden werden kann.

Als Verdünnungsmittel bei dem erfindungsgemäßen Verfahren (C-$C_2$/Variante α) alle gegenüber Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Bei der Durchführung des erfindungsgemäßen Verfahrens (C-$C_2$)/Variante α) werden die Ausgangsstoffe der Formeln (Ib) und (VI) im allgemeinen in angenähert äquivalenten Mengen verwendet. Es ist jedoch auch möglich, die eine oder andere Komponente in einem größeren Überschuß einzusetzen. Die Aufarbeitung erfolgt danach nach üblichen Methoden. Im allgemeinen geht man so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch absaugt oder mit einem Wasser wenig mischbaren organischen Lösungsmittel extrahiert, die organische Phase wäscht, einengt und den verbleibenden Rückstand gegebenenfalls üblichen Reinigungsverfahren unterwirft.

Die Reaktionstemperaturen können bei der Durchführung des erfindungsgemäßen Verfahrens (C-$C_2$/Variante α) innerhalb eines größeren Bereiches variiert werden. Arbeitet man in Gegenwart eines Verdünnungsmittels und eines Säurebindemittels, so liegen die Reaktionstemperaturen im allgemeinen zwischen -20 °C und +100 °C, vorzugsweise zwischen 0 °C und 50 °C.

Die bei dem erfindungsgemäßen Verfahren (C-$C_2$/Variante β) als Reaktionskomponenten benötigten symmetrischen Carbonsäure-anhydride sind durch die Formel (VII) eindeutig definiert. In dieser Formel hat $R^9$ vorzugsweise diejenige Bedeutung, die bereits im Zusammenhang mit der Beschreibung der erfindungsgemäßen Stoffe der Formel (I) vorzugsweise für diesen Rest genannt wurde.

Die symmetrischen Carbonsäureanhydride der Formel (VII) sind bekannt oder lassen sich nach im Prinzip bekannten Methoden in einfacher Weise herstellen.

Als Verdünnungsmittel können bei der Durchführung des erfindungsgemäßen Verfahrens (C-$C_2$/Variante β) vorzugsweise diejenigen Verdünnungsmittel verwendet werden, die auch bei dem Verfahren (C-$C_2$/Variante α) vorzugsweise in Betracht kommen. Im übrigen kann auch ein im Überschuß eingesetztes Carbonsäureanyhdrid der Formel (VII) gleichzeitig als Verdünnungsmittel fungieren.

Die Reaktionstemperaturen können auch bei dem erfindungsgemäßen Verfahren (C-$C_2$/Variante β) innerhalb eines größeren Bereiches variiert werden. Im allgemeinen arbeitet man bei Temperaturen zwischen -20 °C und +150 °C, vorzugsweise zwischen 0 °C und 100 °C.

Bei der Durchführung des erfindungsgemäßen Verfahrens (C-$C_2$/Variante β) werden die Ausgangsstoffe der Formeln (Ib) und (VII) im allgemeinen in angenähert äquivalenten Mengen verwendet. Es ist jedoch auch möglich, das Carbonsäureanhydrid in einem größeren Überschuß einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Die bei dem erfindungsgemäßen Verfahren (C-$C_2$/Variante γ) als Reaktionskomponenten benötigten asymmetrischen Säureanhydride sind durch die Formel (VIII) eindeutig definiert. In dieser Formel hat $R^9$ vorzugsweise diejenige Bedeutung, die bereits im Zusammenhang mit der Beschreibung der erfindungsgemäßen Stoffe der Formel (I) vorzugsweise für diesen Rest genannt wurde. R steht vorzugsweise für Alkyl mit 1 oder 2 Kohlenstoffatomen oder für Phenyl.

Die asymmetrischen Säureanhydride der Formel (VIII) sind bekannt oder lassen sich nach im Prinzip bekannten Methoden in einfacher Weise herstellen. So erhält man Verbindungen der Formel (VIII) dadurch, daß man Carbonsäure der Formel

$$HO-\overset{\overset{O}{||}}{C}-R^9$$

in welcher
$R^9$ die oben angegebene Bedeutung hat,
mit Kohlensäureesterchloriden der Formel (XV)

$$R-O-\overset{\overset{O}{||}}{C}-Cl \quad (XV)$$

in welcher
R die oben angegebene Bedeutung hat,
in Gegenwart eines Verdünnungsmittels, wie z.B. Methylenchlorid, und in Gegenwart eines Säurebindemittels, wie z.B. Triethylamin, bei Temperaturen zwischen -20° C und +100° C, vorzugsweise zwischen 0° C und 50° C, umsetzt.

Die asymmetrischen Säureanhydride der Formel (VIII) werden im allgemeinen nicht in reiner Form isoliert, sondern in der anfallenden Form, gegebenenfalls nach vorheriger Entfernung von Verdünnungsmittel und/oder Salzen, weiter verarbeitet.

Als Verdünnungsmittel können bei der Durchführung des erfindungsgemäßen Verfahrens (C-$C_2$/Variante $\gamma$) vorzugsweise diejenigen Verdünnungsmittel verwendet werden, die auch bei dem Verfahren (C-$C_2$/Variante $\alpha$) vorzugsweise in Betracht kommen. Im übrigen kann auch im Überschuß eingesetztes Säureanhydrid der Formel (VIII) gleichzeitig als Verdünnungsmittel fungieren.

Die Reaktionstemperaturen können auch bei dem erfindungsgemäßen Verfahren (C-$C_2$/Variante $\gamma$) innerhalb eines größeren Bereiches variiert werden. Im allgemeinen arbeitet man bei Temperaturen zwischen -20° C und +150° C, vorzugsweise zwischen 0° C und 100° C.

Bei der Durchführung des erfindungsgemäßen Verfahrens (C-$C_2$/Variante $\gamma$) werden die Ausgangsstoffe der Formeln (Ib) und (VIII) im allgemeinen in angenähert äquivalenten Mengen verwendet. Es ist jedoch auch möglich, das Säureanhydrid in einem größeren Überschuß einzusetzen. Die Aufarbeitung erfolgt nach üblichen Methoden.

Die als Reaktionskomponenten benötigten $\omega$-Halogencarbonsäurehalogenide (Verfahren C-$C_2$/Variante $\delta$) sind durch die Formel (IX) eindeutig definiert. In dieser Formel haben $R^{12-1}$, $R^{12-2}$, $R^{12-3}$, $R^{12-4}$ und R vorzugsweise diejenigen Bedeutungen, die bereits im Zusammenhang mit der Beschreibung der erfindungsgemäßen Stoffe der Formel (I) vorzugsweise für diese Reste genannt wurden. Hal und $Hal^2$ stehen vorzugsweise für Chlor und Brom.

Die $\omega$-Halogencarbonsäurehalogenide der Formel (IX) sind bekannt oder lassen sich nach im Prinzip bekannten Methoden in einfacher Weise herstellen.

Als Säurebindemittel kommen bei der Umsetzung nach dem erfindungsgemäßen Verfahren (C-$C_2$/Variante $\delta$) alle üblichen Säureakzeptoren in Betracht. Für die 1. Stufe verwendet man vorzugsweise tert. Amine, wie Triethylamin, Pyridin und N,N-Dimethylanilin, ferner Alkali- und Erdalkalimetalloxide wie Natriumcarbonat, Kaliumcarbonat und Calciumcarbonat.

Für den 2. Reaktionsschritt werden vorzugsweise Erdalkalihydroxide wie Kaliumhydroxid und Natriumhydroxid, ferner Alkali- oder Erdalkalialkoholate wie Natriummethanolat, Magnesiumethanolat und Kalium-tert.-butylat sowie Alkali- oder Erdalkalihydride wie Natriumhydrid, Kaliumhydrid und Calciumhydrid verwendet.

Als Verdünnungsmittel können bei dem erfindungsgemäßen Verfahren (C-$C_2$/Variante $\delta$) alle gegenüber Säurehalogeniden und starken Basen inerten Solventien eingesetzt werden. Vorzugsweise verwendbar sind Kohlenwasserstoffe wie Benzol, Toluol, Xylol und Tetralin, weiterhin Ether wie Diethylether, Tetrahydrofuran und Dioxan sowie polare Solventien wie Dimethylsulfoxid, Sulfolan, N-Methylpyrrolidon oder Dimethylformamid.

Bei der Durchführung des erfindungsgemäßen Verfahrens (C-$C_2$/Variante $\delta$) werden die Ausgangsstoffe der Formeln (Ib) und (IX) im allgemeinen in angenähert äquivalenten Mengen verwendet. Es ist jedoch auch möglich, die eine oder andere Komponente in einem größeren Überschuß einzusetzen. Die Aufarbeitung erfolgt nach den üblichen Methoden.

Die Reaktionstemperaturen können bei der Durchführung des erfindungsgemäßen Verfahrens (C-$C_2$/Variante $\delta$) innerhalb eines größeren Bereiches variiert werden. Arbeitet man in Gegenwart eines Verdünnungsmittels und in Gegenwart eines Säurebindemittels, so liegen die Reaktionstemperaturen im allgemeinen zwischen -78° C und +100° C, vorzugsweise zwischen -78° C und +50° C.

Die erfindungsgemäßen Verfahren (C-$C_2$/Variante α, β, γ und δ) werden im allgemeinen bei Normaldruck durchgeführt.

Die zur Durchführung des erfindungsgemäßen Verfahrens (D) als Ausgangsstoffe benötigten Verbindungen der Formel (Ic) sind erfindungsgemäße Stoffe und erhältlich nach den Verfahren (A), (B) oder (C).

In der Formel (IC) haben $R^1$, $R^2$, $R^3$, $R^4$, $R^{5-1}$, $R^6$ und X vorzugsweise diejenigen Bedeutungen, die bereits bei der Beschreibung der erfindungsgemäßen Stoffe der Formel (I) vorzugsweise für diese Substituenten genannt wurden, $R^{5-1}$ steht für den Rest 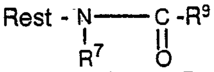 , wobei $R^7$ und $R^9$ vorzugsweise für die Reste, die bei der Beschreibung der Stoffe der Formel (I) vorzugsweise genannt wurden.

Als Verdünnungsmittel zur Durchführung des erfindungsgemäßen Verfahrens (D) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan oder Tetrahydrofuran.

Die Reaktionstemperaturen können bei der Durchführung des erfindungsgemäßen Verfahrens (D) innerhalb eines größeren Bereiches variiert werden. Im allgemeinen arbeitet man bei Temperaturen zwischen +20 °C und +200 °C, vorzugsweise zwischen 50 °C und 150 °C.

Das erfindungsgemäße Verfahren (D) wird im allgemeinen unter Normaldruck durchgeführt. Es ist jedoch auch möglich bei erhöhtem oder vermindertem Druck zu arbeiten.

Zur Durchführung des erfindungsgemäßen Verfahrens (D) setzt man pro Mol an Verbindung der Formel (Ic) 0,5 bis 5,0 Mol, vorzugsweise 0,5 bis 2,0 Mol, an Schwefelungsagentien ein.

In den bei dem erfindungsgemäßen Verfahren (E) als Ausgangsstoffe benötigten Verbindungen der Formel (Id) stehen $R^1$, $R^2$, $R^3$, $R^4$, $R^{5-2}$, $R^6$ und X vorzugsweise für diejenigen Bedeutungen, die bereits bei der Beschreibung der erfindungsgemäßen Stoffe der Formel (I) vorzugsweise für diese Substituenten bzw. diesen Index genannt wurden. Die Verbindungen der Formel (Id) sind erfindungsgemäße Verbindungen und erhältlich nach Verfahren (D).

Die bei dem erfindungsgemäßen Verfahren (E) weiterhin als Ausgangsstoffe benötigten Verbindungen sind durch die Formel (X) allgemein definiert. In der Formel (X) hat $R^{11}$ vorzugsweise diejenige Bedeutung, die bereits bei der Beschreibung der erfindungsgemäßen Stoffe der Formel (I) vorzugsweise für diesen Substituenten genannt wurde. L steht vorzugsweise für Chlor, Brom, Methylsulfonyloxy, Ethylsulfonyloxy oder Phenylsulfonyloxy.

Die Verbindungen der Formel (X) sind allgemein bekannte Verbindungen der organischen Chemie.

Als deprotonierende Basen zur Durchführung des erfindungsgemäßen Verfahrens (E) kommen alle üblichen für derartige Reaktionen verwendbaren Basen infrage.

Vorzugsweise verwendet man Alkalialkoholate wie beispielsweise Kalium-tert.-butylat, Natriummethylat, Natriumbutylat, Natrium- oder Kaliumhydroxid, Kaliumcarbonat, Natrium- oder Lithiumhydrid.

Als Verdünnungsmittel zur Durchführung des erfindungsgemäßen Verfahrens (E) kommen inerte organische Lösungsmittel infrage. Vorzugsweise verwendet man Alkohole wie beispielsweise tert.-Butanol, Ethanol, Isopropanol, Dimethylformamid, N-Methylpyrrolidon, Dimethylsulfoxid, Sulfolan, Tetrahydrofuran oder Dioxan.

Die Reaktionstemperaturen können bei der Durchführung des erfindungsgemäßen Verfahrens innerhalb eines größeren Bereiches variiert werden. Im allgemeinen arbeitet man bei Temperaturen zwischen 0 °C und 200 °C, vorzugsweise bei 20 °C bis 120 °C.

Das erfindungsgemäße Verfahren (E) wird im allgemeinen bei Normaldruck durchgeführt. Es ist jedoch auch möglich, bei erhöhtem oder vermindertem Druck zu arbeiten.

In den bei dem erfindungsgemäßen Verfahren (F) als Ausgangsstoffe benötigten Verbindungen der Formel (If) stehen $R^1$, $R^2$, $R^3$, $R^4$, $R^6$ und X vorzugsweise für diejenigen Bedeutungen, die bereits bei der Beschreibung der erfindungsgemäße Stoffe der Formel (I) vorzugsweise für diese Substituenten genannt wurden. $R^{5-3}$ steht für Hydroxy, Verbindungen der Formel (If) sind erfindungsgemäße Verbindungen und erhältlich nach Verfahren (A).

Die bei dem erfindungsgemäßen Verfahren (F) weiterhin als Ausgangsstoffe benötigten Verbindungen sind durch die Formel (XI) allgemein definiert. In der Formel (XI) hat $R^{5-4}$ vorzugsweise diejenige Bedeutung, die bereits bei der Beschreibung der Stoffe der Formel (I) vorzugsweise für die entsprechenden Substituenten genannt wurde. G steht vorzugsweise für Chlor, Brom, Methylsulfonyloxy, Phenylsulfonyloxy oder Tolylsulfonyloxy.

Die Verbindungen der allgemeinen Formel (XI) sind bekannt oder lassen sich nach im Prinzip bekannten Verfahren in einfacher Weise herstellen (vgl. z.B. Helv. Chim. Acta 63 1412 (1980)).

Als Verdünnungsmittel können bei dem erfindungsgemäßen Verfahren (F) alle inerten Solventien eingesetzt werden. Vorzugsweise verwendbar sind Halogenkohlenwasserstoffe, wie Methylenchlorid, Chloroform, Tetrachlorkohlenstoff, Chlorbenzol und o-Dichlorbenzol und Kohlenwasserstoffe wie Toluol, Xylol und Tetralin, weiterhin cyclische Ether, wie Tetrahydrofuran und Dioxan, darüber hinaus Carbonsäureester, wie Ethylacetat und Glykolmonomethyletheracetat und auch stark polare Solventien, wie Dimethylsulfoxid, Sulfolan, N-Methylpyrrolidon, Dimethylacetamid und Dimethylformamid.

Als Säurebindemittel kommen bei der Umsetzung nach dem erfindungsgemäßen Verfahren (F) alle üblichen Säureakzeptoren in Betracht. Vorzugsweise verwendbar sind tertiäre Amine, wie Triethylamin, Pyridin und N,N-Di methyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat, außerdem Alkali- und Erdalkali-metallhydroxide, wie Lithiumhydroxid, Natriumhydroxid, Kaliumhydroxid und Calciumhydroxid, Alkali- und Erdalkalialkoholate wie Natriummethylat, Natriumethylat, Kalium-tert.-butylat und Magnesiumethylat, Alkali- und Erdalkalihydride wie Natriumhydrid, Kaliumhydrid und Calciumhydrid.

Die Reaktionstemperaturen können bei der Durchführung des erfindungsgemäßen Verfahrens (F) innerhalb eines größeren Bereiches variiert werden. Im allgemeinen arbeitet man bei Temperaturen zwischen -20° C und +250° C, vorzugsweise zwischen 50° C und 200° C.

Bei der Durchführung des erfindungsgemäßen Verfahrens (F) werden die Ausgangsstoffe der Formeln (If) und (XI) und auch gegebenenfalls die Base im allgemeinen in angenähert äquivalenten Mengen verwendet. Es ist jedoch auch möglich die eine oder andere Reaktionskomponente in einem größeren Überschuß einzusetzen. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B.

auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfen-anlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono-und dikotyler Unkräuter insbesondere in monokotylen Kulturen im Nachauflaufverfahren einsetzen.

Darüberhinaus zeigen die erfindungsgemäßen Wirkstoffe eine wuchsregulierende Wirkung und können als Defoliantmittel verwendet werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide, Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit Aryloxyalkansäuren wie 2,4D; 2,4DP; 2,4DB; MCPA; MCPP oder Triclopyr; Aryloxy-phenoxyalkansäureester wie Diclofop-methyl, Fenoxaprop, (2R)-2-[4-(3,5-Dichlor-pyridyl-2-oxy)-phenoxy]-propionsäure-(trimethylsilyl)-methylester; Arylcarbonsäuren wie Dichlorpicolinsäure; Diphenylether wie Bifenox; Harnstoffe wie Chlortoluron, Fluormeturon, Isoproturon, Methabenzthiazuron; Imidazolinone wie Imazamethabenz; Nitrile wie Bromoxynil oder Ioxynil; Oxyacetamide wie Mefenacet; Sulfonylharnstoffe wie Chlorsulfuron, Thiameturon; Thiolcarbamate wie Triallate, Triazindione wie Amethydione; Triazine wie Terbutryne, Triazinone wie Ethozin oder Metribuzin sowie Bentazone kommen infrage. Einige Mischungen

zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel I-1 (Verfahren A)

2-(4-Neopentyloxyphenylthio)-4-methylamino-6-methylpyrimidin

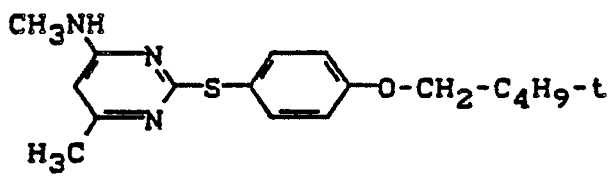

3,53 g (20 mMol) 4-Neopentyloxy-thiophenol werden in 20 ml N-Methylpyrrolidon unter Stickstoffatmosphäre vorgelegt und mit 1,23 g (22 mMol) gepulvertem Kaliumhydroxid versetzt. Nach 15 Minuten werden 3,62 g (20 mMol) 4-N-Methylamino-6-methyl-2-methylsulfonylpyrimidin hinzugefügt und 2 Stunden auf 120 °C erwärmt. Die Reaktionsmischung wird in 150 ml 1N NatriumhydroxidLösung eingerührt, der Niederschlag wird abgesaugt und getrocknet. Durch Umkristallisieren aus Toluol/n-Hexan erhält man 4,63 g (81 %) 2-(4-Neopentyloxyphenylthio)-4-methylamino-6-methylpyrimidin vom Schmelzpunkt 104 °C.

### Beispiel I-2 (Verfahren B)

2-(4-Nitrophenylthio)-4-amino-6-methylpyrimidin

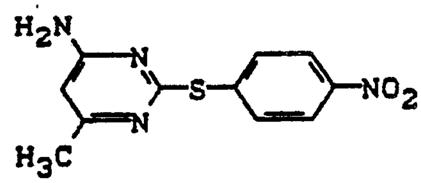

28,2 g (0,1 Mol) 2-(4-Nitro-phenylthio)-4-chlor-6-methyl-pyrimidin werden in 200 ml Dioxan in einem VA-Druckautoklaven mit 5,1 g (0,3 Mol) flüssigem Ammoniak versetzt. Die Mischung wird 5 Stunden auf 120 bis 125 °C erhitzt (Innendruck bis 37 bar). Nach dem Entspannen wird das Lösungsmittel weitgehend abdestilliert, der Rückstand in 1 Liter Wasser eingerührt. Das ausgefallene Produkt wird abgesaugt, mit Wasser gewaschen und an der Luft getrocknet. Man erhält etwa 25 g (95 % der Theorie) 2-(4-Nitrophenylthio)-4-amino-6-methyl-pyrimidin vom Schmelzpunkt 175 bis 177 °C (aus Butanol umkristallisiert).

Beispiel I-3 (Verfahren B)

2-(4-Nitrophenylthio)-4-methylamino-6-methylpyrimidin

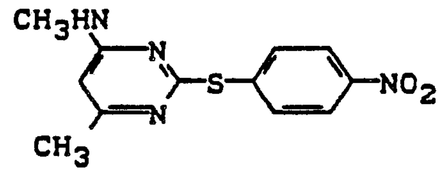

28,2 g (0,1 Mol) 2-(4-Nitro-phenylthio)-4-chlor-6-methyl-pyrimidin werden in 200 ml Dioxan gelöst. In diese Lösung leitet man unter Rühren anfangs bei Raumtemperatur, dann bei bis zu 75 °C steigender Temperatur gasförmiges Methylamin im Überschuß (ca. 10 g ~ 0,3 Mol) ein. Wenn durch dünnschichtchromatographischen Nachweis das Vorprodukt vollständig umgesetzt ist, dampft man das Lösungsmittel im Vakuum weitgehend ab und verrührt den Rückstand mit Wasser. Das Festprodukt wird abgesaugt, mit Wasser gewaschen und an der Luft getrocknet. Man gewinnt 25,8 g (93,5 % der Theorie) 2-(4-Nitrophenylthio)-4-methylamino-6-methyl-pyrimidin vom Schmelzpunkt Fp: 130 °C bis 132 °C (aus Tetrachlormethan umkristallisiert).

Beispiel I-4 (Verfahren C-$C_1$)

2-(4-Aminophenylthio)-4-amino-6-methylpyrimidin

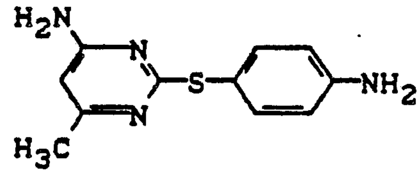

26,2 g (0,2 Mol) 2-(4-Nitro-phenylthio)-4-amino-6-methyl-pyrimidin werden in 250 ml Dioxan gelöst und nach Zugabe von 3 g Raney-Nickel in einem VA-Druckautoklaven bis zur Sättigung hydriert (4 Stunden).

Dabei steigt die Temperatur bis auf 55 °C an. Nach Entspannen des Autoklaven wird der Katalysator abfiltriert, die Lösung im Vakuum eingedampft. Man erhält 22,5 g (97 % der Theorie) 2-(4-Amino-phenylthio)-4-amino-6-methyl-pyrimidin vom Schmelzpunkt Fp. 176 °C bis 178 °C (aus Butanol umkristallisiert).

Beispiel I-5 (Verfahren C-C2/Variante α)

2-(4-Pivaloylaminophenylthio)4-amino-6-methylpyrimidin

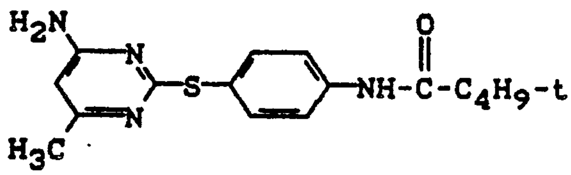

23,2 g (0,1 Mol) 2-(4-Amino-phenylthio)-4-amino-6-methyl-pyrimidin werden in 200 ml Tetrahydrofuran gelöst. Hierzu gibt man 10,1 g (0,1 Mol) Triethylamin und tropft anschließend unter schwacher Kühlung bei 15 °C bis 20 °C 12,05 g (0,1 Mol) Pivalsäurechlorid zu. Die Mischung wird noch 3 Stunden bei Raumtemperatur nachgerührt, im Vakuum eingedampft und der Rückstand mit 1 Liter Wasser verrührt. Die abgeschiedenen Kristalle werden abgesaugt, mit Wasser gewaschen und an der Luft getrocknet. Man erhält 29 g (91,7 % der Theorie) 2-(4-Pivaloylamino-phenylthio)-4-amino-6-methyl-pyrimidin vom Schmelzpunkt Fp: 182 °C bis 184 °C. Nach Befund des $^1H$-NMR bleibt die 4-Aminogruppe im Pyrimidinring unter diesen Bedingungen unverändert.

Beispiel I-6 (Verfahren C-C2/Variante δ)

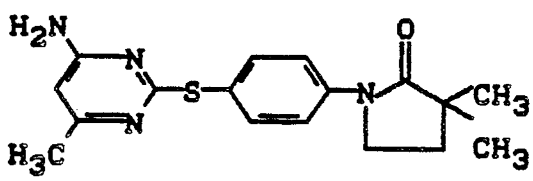

23,2 g (0,1 Mol) 4-(4-Amino-6-methyl-2-pyrimidylthio)-anilin werden in 300 ml absolutem Tetrahydrofuran gelöst und mit 10,2 ml (0,1 Mol) absolutem Pyridin versetzt. Bei 0 °C bis 10 °C werden 21,11 g (0,105 Mol) 4-Chlor-2,2-dimethyl-buttersäurechlorid zugetropft und anschließend 30 Minuten bei Raumtemperatur gerührt. Nach Absaugen des Hydrochlorids engt man das Filtrat auf ca. 100 ml ein und gibt dieses schnell zu einer Suspension aus 12,33 g (0,11 Mol) Kalium-tert.-butylat in 80 ml absolutem Tetrahydrofuran, die auf -70 °C vorgekühlt ist. Die Mischung wird in 1 Liter Eiswasser eingerührt, der Niederschlag abgesaugt, getrocknet und aus Chloroform/n-Hexan umkristallisiert. Man erhält 13,72 g (41,8 % der Theorie) des obengenannten Pyrrolidons vom Schmelzpunkt 208 °C.

In entsprechender Weise und gemäß den allgemeinen Angaben zur Herstellung erhält man die in der folgenden Tabelle aufgeführten Aryloxy (bzw. thio)aminopyrimidine der Formel (I)

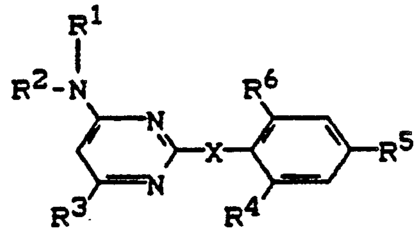

(I)

(Fp. = Schmelzpunkt)

Tabelle 1

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 7 | $-CH_3$ | $-CH_3$ | $-CH_3$ | S | -H | -H | $-CH_3$ | 90 |
| 8 | 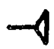 | -H | $-CH_3$ | S | -H | -H | $-CH_3$ | Öl |
| 9 | $-C_3H_7$-i | -H | $-CH_3$ | S | -H | -H | $-CH_3$ | Öl |
| 10 | $-C_4H_9$-t | -H | $-CH_3$ | S | -H | -H | $-CH_3$ | 155 |
| 11 | -H | -H | $-CH_3$ | S | -H | -H | $-CH_3$ | 104 |
| 12 | -H | -H | $-CH_3$ | S | -H | -H | $-CH_3$ | 154 |
| 13 | -H | -H | $-CH_3$ | S | -H | -H | -Cl | 163 |
| 14 | -H | -H | $-CH_3$ | O | -H | -H | $-CH_3$ | 79 |
| 15 | -H | -H | $-CH_3$ | O | -H | -H | $-C_2H_5$ | 90 |
| 16 | -H | -H | $-CH_3$ | S | -H | -H | $-C_4H_9$-t | 130 |
| 17 | -H | -H | $-CH_3$ | O | -H | -H | $-OCH_3$ | 184 |
| 18 | -H | -H | $-CH_3$ | S | -H | -H | -F | 121 |
| 19 | -H | -H | $-CH_3$ | O | -H | -H | $-OCF_3$ | |
| 20 | -H | -H | $-CH_3$ | S | -H | -H | $-OCH_3$ | 180 |
| 21 | -H | -H | $-CH_3$ | O | -H | -H | -F | 140 |
| 22 | -H | -H | $-CH_3$ | O | -H | -H | $-OC_3H_7$-i | 112 |
| 23 | -H | -H | $-CH_3$ | O | -H | -H | $-CF_3$ | 152 |
| 24 | -H | -H | $-CH_3$ | O | -H | -H | -Cl | 119 |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 25 | -H | -H | $-CH_3$ | O | -H | -H | $-OC_2H_5$ | |
| 26 | $-C_2H_5$ | -H | $-CH_3$ | S | -H | -H | $-O-CH_2-C_4H_9-t$ | 108 |
| 27 | $-CH_3$ | $-CH_3$ | $-CH_3$ | S | -H | -H | $-O-CH_2-C_4H_9-t$ | 72 |
| 28 | $-C_3H_7-i$ | -H | $-CH_3$ | S | -H | -H | $-O-CH_2-C_4H_9-t$ | 128 |
| 29 | -H | -H | $-CH_3$ | S | -H | -H | $-O-CH_2-C_4H_9-t$ | 114 |
| 30 | $-CH_3$ | -H | $-CH_3$ | S | -H | -H | $-(CH_2)_2-C_4H_9-t$ | 105 |
| 31 | -H | -H | $-CH_3$ | S | -H | -H | $-(CH_2)_2-C_4H_9-t$ | 111 |
| 32 | -H | -H | $-CH_3$ | S | $-CH_3$ | -H | $-O-CH_2-C_4H_9-t$ | 129 |
| 33 | $-CH_3$ | $-CH_3$ | $-CH_3$ | O | -Cl | -H | -H | 79 |
| 34 | $-CH_2-CH=CH_2$ | -H | $-CH_3$ | S | -H | -H | $-O-CH_2-C_4H_9-t$ | 99 |
| 35 | $-CH_3$ | -H | $-CH_3$ | S | -Cl | -H | $-O-CH_2-C_4H_9-t$ | 129 |
| 36 | -H | -H | $-CH_3$ | S | -Cl | -H | $-O-CH_2-C_4H_9-t$ | 77 |
| 37 | -H | -H | $-CH_3$ | S | -H | -H | $-O-CH-C_4H_9-t$<br>\|<br>$CH_3$ | 151 |
| 38 | -H | -H | $-CH_3$ | S | -H | -H | $(-CH_2)_2-C_3H_7-i$ | 97 |
| 39 | $-CH_3$ | -H | $-CH_3$ | S | -H | -H | $(-CH_2)_2-C_3H_7-i$ | 83 |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 40 | $-CH_3$ | $-CH_3$ | $-CH_3$ | S | -H | -H | $-NO_2$ | 120-122 |
| 41 | $-CH_3$ | $-CH_3$ | $-CH_3$ | S | -H | -H | $-NH-CO-C_4H_9-t$ | 188-190 |
| 42 | $-CH_3$ | -H | $-CH_3$ | S | -H | -H | $-NH-CO-C_4H_9-t$ | 182-184 |
| 43 | $-C_2H_5$ | $-C_2H_5$ | $-CH_3$ | S | -H | -H | $-NH-CO-C_4H_9-t$ | 146-148 |
| 44 | $-C_3H_7-i$ | -H | $-CH_3$ | S | -H | -H | $-NO_2$ | 98-100 |
| 45 | $-C_3H_7-i$ | -H | $-CH_3$ | S | -H | -H | $-NH_2$ | 142-144 |
| 46 | $-C_3H_7-i$ | -H | $-CH_3$ | S | -H | -H | $-NH-CO-C_4H_9-t$ | 75 (Zers.) |
| 47 | $-C_2H_5$ | -H | $-CH_3$ | S | -H | -H | $-NO_2$ | 104-106 |
| 48 | $-C_2H_5$ | -H | $-CH_3$ | S | -H | -H | $-NH_2$ | 148-150 |
| 49 | $-C_2H_5$ | -H | $-CH_3$ | S | -H | -H | $-NH-CO-C_4H_9-t$ | 144-146 |
| 50 | $-CH_3$ | -H | $-CH_3$ | S | -H | -H | $-NH_2$ | 198 |
| 51 | -H | -H | $-CH_3$ | S | -H | -H | $-NH-CO-C_3H_7-i$ | 116-117 |
| 52 | -H | -H | $-CH_3$ | S | -H | -H | $-NH-CO-C_2H_5$ | 191-192 |
| 53 | -H | -H | $-CH_3$ | S | -H | -H | $-NH-CO-CH_2-OCH_3$ | 154-156 |
| 54 | -H | -H | $-CH_3$ | S | -H | -H | $-NH-CO-CH_3$ | 214-216 |
| 55 | -H | -H | $-CH_3$ | S | -H | -H | -NH-CO- 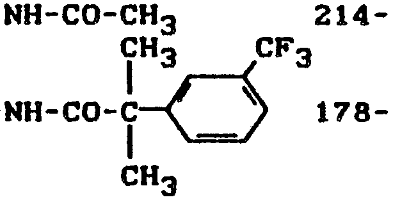 | 178-180 |

Tabelle 1 (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 56 | -H | -H | $-CH_3$ | S | -H | -H | $-NH-CO-CH_2-C_4H_9$-t | 189-191 |
| 57 | -H | -H | $-CH_3$ | S | $-CH_3$ | -H | $-NO_2$ | 187-189 |
| 58 | -H | -H | $-CH_3$ | S | -H | -H | -NH-CO 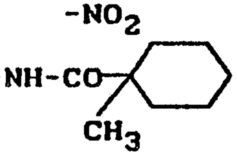 | 196-198 |
| 59 | -H | -H | $-CH_3$ | S | -H | -H | 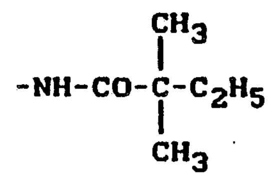 | 182-184 |
| 60 | -H | -H | $-CH_3$ | S | $-OCH_3$ | -H | $-NO_2$ | 192-193 |
| 61 | -H | -H | $-CH_3$ | S | $-OCH_3$ | -H | $-NH_2$ | 179-181 |
| 62 | -H | -H | $-CH_3$ | S | $-OCH_3$ | -H | 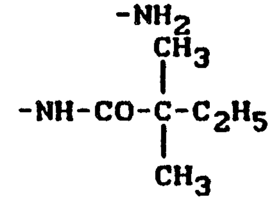 | 176-178 |
| 63 | -H | -H | $-CH_3$ | S | $-CH_3$ | -H | $-NH_2$ | 162-164 |
| 64 | $-CH_3$ | $-CH_3$ | $-CH_3$ | S | -H | -H | $-NH_2$ | 149-151 |

Tabelle 1 (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 65 | $CH_3$ | $CH_3$ | $CH_3$ | S | -H | -H | $-(CH_2)_2-C_3H_7-i$ | 46 |
| 66 | $CH_3$ | $CH_3$ | $CH_3$ | S | -H | -H | $-(CH_2)_2-C_4H_9-t$ | 61 |
| 67 | $CH_3$ | H | $CH_3$ | S | -H | -H | $-O-(CH_2)_2-C_4H_9-t$ | 116 |
| 68 | $CH_3$ | H | $CH_3$ | S | -H | -H | 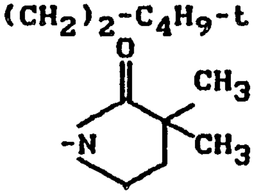 | 111 |
| 69 | $CH_3$ | $CH_3$ | $CH_3$ | S | -H | -H | 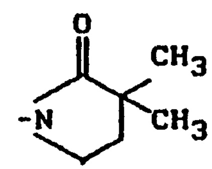 | 123 |
| 70 | H | H | $CH_3$ | S | $CH_3$ | -H | 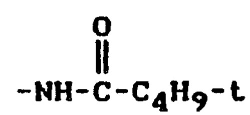 | 236-237 |
| 71 | H | H | $CH_3$ | S | $CH_3$ | -H | 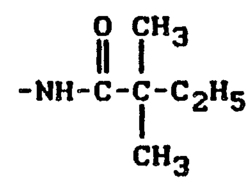 | 208-210 |

<u>Tabelle 1</u> (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 72 | H | H | $CH_3$ | S | H | -H | $-NH-C(=O)-OC_2H_5$ | 210-212 |
| 73 | H | H | $CH_3$ | S | H | -H | $-NH-C(=O)-CH(CH_3)-CH_2-OCH_3$ | 78- 80 |
| 74 | H | H | $C_2H_5$ | S | H | -H | $-NH-C(=O)-C_4H_9$-t | 184 |
| 75 | H | H | $CH_3$ | S | H | -H | $-NH-C(=O)-C(CH_3)=CH-SCH_3$ | 190-192 |
| 76 | H | H | $C_3H_7$ | S | H | -H | $-NH-C(=O)-C_4H_9$-t | 183-185 |

Tabelle 1 (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 77 | H | H | $C_3H_7$ | S | H | -H | 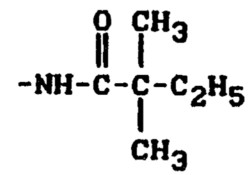 | 84- 86 |
| 78 | H | H | $C_4H_9$ | S | H | -H | 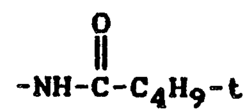 | |
| 79 | $CH_3$ | H | $CH_3$ | S | H | -H | -OH | 211-217 |
| 80 | $CH_3$ | $CH_3$ | $CH_3$ | S | H | -H | -OH | 218-221 |
| 81 | H | H | $CH_3$ | S | H | H | 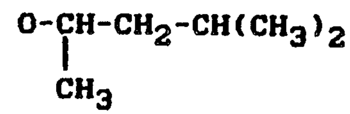 | 92 |
| 82 | H | H | $CH_3$ | S | H | H | 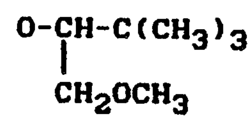 | 78 |
| 83 | H | H | $CH_3$ | S | H | H | $O-(CH_2)_2-C(CH_3)_2OCH_3$ | 111 |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 84 | H | H | $CH_3$ | S | H | H | $O-(CH_2)_2-$ 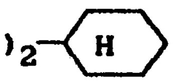 | 129 |
| 85 | H | H | $CH_3$ | S | H | H | $O-(CH_2)_2-CH(CH_3)_2$ | 118 |
| 86 | H | H | $CH_3$ | S | H | H | $O-CH_2-C(CH_3)_2-CH_2-O-CH_3$ | 143 |
| 87 | H | H | $CH_3$ | S | H | H | $O-CH_2-C(CH_3)_2-OC_2H_5$ | 92 |
| 88 | H | H | $CH_3$ | S | H | H | $O-(CH_2)_2-C(CH_3)_2-OC_2H_5$ | 78 |
| 89 | H | H | $CH_3$ | S | H | H | $O-CH_2-C(CH_3)_2-OCH_3$ | 110 |
| 90 | H | H | $CH_3$ | S | H | H | $O-CH_2-C(CH_3)_2-CH_2Cl$ | 115 |
| 91 | H | H | $CH_3$ | S | H | H | $O-CH_2-CH(CH_3)_2$ | 92 |
| 92 | H | H | $CH_3$ | S | H | H | $-O-(CH_2)_2C(CH_3)_2$ | 108 |
| 93 | H | H | $CH_3$ | S | H | H | $O-CH_2-C(CH_3)_2-CH_2-O-C_2H_5$ | Öl |
| 94 | H | H | $CH_3$ | S | H | H | $O-(CH_2)_2C(CH_3)_2C_2H_5$ | 60 |
| 95 | H | H | $CH_3$ | S | H | H | $O-CH_2-C(CH_2-OCH_3)_2CH_3$ | 48 |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 96 | H | H | $CH_3$ | S | H | H | $O-CH_2-C(CH_2-O-CH_3)_3$ | 100 |
| 97 | H | H | $CH_3$ | S | $CH_3$ | H | $O-(CH_2)_2C(CH_3)_3$ | 53 |
| 98 | H | H | $CH_3$ | S | Cl | H | $O-(CH_2)_2C(CH_3)_3$ | 38 |
| 99 | $CH_3$ | H | $CH_3$ | S | H | H | $O-CH_2-C(CH_3)_2CH_2F$ | 111 |
| 100 | $CH_3$ | H | $CH_3$ | S | H | H | $O-CH_2-CH(CH_3)_2$ | 90 |
| 101 | $CH_3$ | H | $CH_3$ | S | H | H | $O-CH_2-C(CH_3)_2-CH_2O-CH_3$ | 42 |
| 102 | $CH_3$ | H | $CH_3$ | S | H | H | $O-CH_2-C(CH_3)_2-OCH_3$ | 77 |
| 103 | $CH_3$ | H | $CH_3$ | S | H | H | $O-CH-C(CH_3)_3$<br>\|<br>$CH_2-OCH_3$ | 92 |
| 104 | $CH_3$ | H | $CH_3$ | S | H | H | $O-CH-C(CH_3)_3$<br>\|<br>$C_2H_5$ | 142 |
| 105 | $CH_3$ | H | $CH_3$ | S | H | H | $O-CH-CH_2-CH(CH_3)_2$<br>\|<br>$CH_3$ | 82 |

<u>Tabelle 1</u> (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 106 | $CH_3$ | H | $CH_3$ | S | H | H | $O-CH_2-C(CH_3)_2-CH_2Cl$ | 109 |
| 107 | $CH_3$ | H | $CH_3$ | S | H | H | $O-CH_2-C(CH_3)_2-OC_2H_5$ | 75 |
| 108 | $CH_3$ | H | $CH_3$ | S | H | H | $O-CH_2-C(CH_3)_2-CH_2-OC_2H_5$ | 76 |
| 109 | $CH_3$ | H | $CH_3$ | S | H | H | $O-CH_2-CH_2-CH(CH_3)_2$ | 85 |
| 110 | $CH_3$ | H | $CH_3$ | S | H | H | 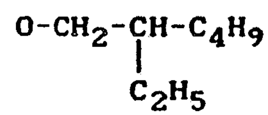 | 73 |
| 111 | $CH_3$ | H | $CH_3$ | S | H | H | 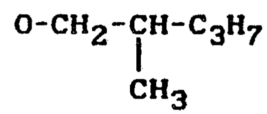 | 66 |
| 112 | $CH_3$ | H | $CH_3$ | S | H | H | $O-(CH_2)_2-C(CH_3)_2OCH_3$ | 85 |
| 113 | $CH_3$ | H | $CH_3$ | S | H | H | 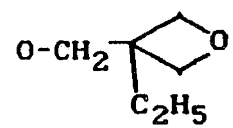 | 70 |
| 114 | $CH_3$ | H | $CH_3$ | S | H | H | 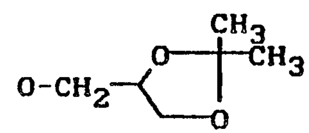 | 110 |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 115 | $CH_3$ | H | $CH_3$ | S | H | H | $O(CH_2)_2-$ 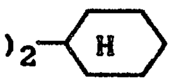 | 193 |
| 116 | $CH_3$ | H | $CH_3$ | S | $CH_3$ | H | $O-(CH_2)_2-C(CH_3)_3$ | 129 |
| 117 | $CH_3$ | H | $CH_3$ | S | Cl | H | $O-(CH_2)_2-C(CH_3)_3$ | 101 |
| 118 | $CH_3$ | $CH_3$ | $CH_3$ | S | H | H | $O-CH_2C(CH_3)_2CH_2F$ | 102 |
| 119 | $CH_3$ | $CH_3$ | $CH_3$ | S | H | H | $O-(CH_2)_2C(CH_3)_3$ | 71 |
| 120 | $CH_3$ | $CH_3$ | $CH_3$ | S | H | H | $O-CH_2CH(CH_3)_2$ | 68 |
| 121 | $CH_3$ | $CH_3$ | $CH_3$ | S | H | H | $O-CH_2-C(CH_3)_2-OCH_3$ | 74 |
| 122 | $CH_3$ | $CH_3$ | $CH_3$ | S | H | H | $O-CH_2-C(CH_3)_2-CH_2-O-CH_3$ | 64 |
| 123 | $CH_3$ | $CH_3$ | $CH_3$ | S | H | H | $O-CH-C(CH_3)_3$<br>$CH_2OCH_3$ | 58 |
| 124 | $CH_3$ | $CH_3$ | $CH_3$ | S | H | H | $O-CH-C(CH_3)_3$<br>$C_2H_5$ | 116 |

Tabelle 1 (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 125 | $CH_3$ | $CH_3$ | $CH_3$ | S | H | H | 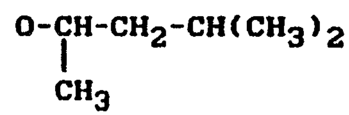 | Öl |
| 126 | $CH_3$ | $CH_3$ | $CH_3$ | S | H | H | $O-CH_2-C(CH_3)_2-CH_2Cl$ | 60 |
| 127 | $CH_3$ | $CH_3$ | $CH_3$ | S | H | H | 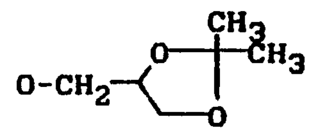 | 95 |
| 128 | $CH_3$ | $CH_3$ | $CH_3$ | S | H | H | $O-CH_2-C(CH_3)_2-CH_2-O-C_2H_5$ | 38 |
| 129 | $CH_3$ | $CH_3$ | $CH_3$ | S | H | H | $O-(CH_2)_2CH(CH_3)_2$ | 48 |
| 130 | $CH_3$ | $CH_3$ | $CH_3$ | S | H | H | 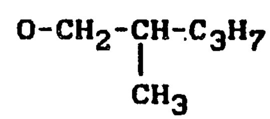 | Öl |
| 131 | $CH_3$ | $CH_3$ | $CH_3$ | S | H | H | $O-CH_2C(CH_3)_2OC_2H_5$ | Öl |
| 132 | $CH_3$ | $CH_3$ | $CH_3$ | S | H | H | 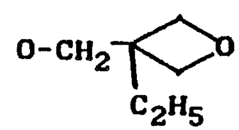 | Öl |
| 133 | $CH_3$ | $CH_3$ | $CH_3$ | S | H | H | $O-(CH_2)_2C(CH_3)_2C_2H_5$ | Öl |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 134 | $CH_3$ | $CH_3$ | $CH_3$ | S | H | H | $O-(CH_2)_2-$ 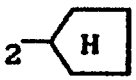 | 50 |
| 135 | $CH_3$ | $CH_3$ | $CH_3$ | S | H | H | $O-(CH_2)_2C(CH_3)_2OC_2H_5$ | Öl |
| 136 | $CH_3$ | $CH_3$ | $CH_3$ | S | $CH_3$ | H | $O-(CH_2)_2C(CH_3)_3$ | 25 |
| 137 | $CH_3$ | $CH_3$ | $CH_3$ | S | Cl | H | $O-(CH_2)_2C(CH_3)_3$ | Öl |
| 138 | H | H | $CH_3$ | S | H | H | $CO-CH_2C(CH_3)_3$ | 123 |
| 139 | H | H | $CH_3$ | S | H | H | $CO-(CH_2)_2-C(CH_3)_3$ | 135 |
| 140 | $CH_3$ | H | $CH_3$ | S | H | H | $CO-CH_2-C(CH_3)_3$ | Öl |
| 141 | $CH_3$ | $CH_3$ | $CH_3$ | S | H | H | $CO-CH_2-C(CH_3)_3$ | 75 |
| 142 | $CH_3$ | $CH_3$ | $CH_3$ | S | H | H | $CO-(CH_2)_2-C(CH_3)_3$ | Öl |
| 143 | H | H | $CH_3$ | S | H | H | $-(CH_2)_3-C(CH_3)_3$ | 77 |
| 144 | $CH_3$ | H | $CH_3$ | S | H | H | $-(CH_2)_3-C(CH_3)_3$ | Öl |
| 145 | $CH_3$ | $CH_3$ | $CH_3$ | S | H | H | $-C(CH_2)_3-C(CH_3)_3$ | Öl |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 146 | H | H | $CH_3$ | S | H | H | $NH\text{-}CO\text{-}CH\text{-}CH_2\text{-}S\text{-}CH_3$<br>\|<br>$CH_3$ | 174-176 |
| 147 | H | H | $CH_3$ | S | H | H | $NH\text{-}CO\text{-}C(CH_3)_2\text{-}CH_2\text{-}O\text{-}CH_3$ | 178-180 |
| 148 | H | H | $CH_3$ | S | H | H | $NH\text{-}CO\text{-}C(CH_3)_2\text{-}CH_2\text{-}O\text{-}CH(CH_3)_2$ | 129-131 |
| 149 | H | H | $CH_3$ | S | H | H | $NH\text{-}CO\text{-}C(CH_3)_2\text{-}CH_2\text{-}O\text{-}C_2H_5$ | 108-110 |
| 150 | H | H | $CH_3$ | S | H | H | $NH\text{-}CO\text{-}C(CH_2OCH_3)_2CH_3$ | 140-142 |
| 151 | H | H | $CH_3$ | S | H | H | $NH\text{-}CO\text{-}C(CH_2OCH_3)_3$ | 118-120 |
| 152 | H | H | $CH_3$ | S | H | H | $NH\text{-}CO\text{-}C(CH_3)_2CH_2\text{-}O\text{-}(CH_2)_2\text{-}O\text{-}CH_3$ | 171 |
| 153 | H | H | $CH_3$ | S | H | H | 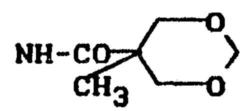 | 217 |
| 154 | H | H | $CH_3$ | S | H | H | 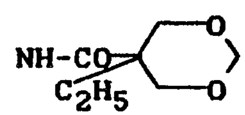 | 157-159 |
| 155 | H | H | $CH_3$ | S | H | H | $NH\text{-}CO\text{-}C(CH_2\text{-}O\text{-}C_2H_5)_2CH_3$ | 80-82 |

**Tabelle 1** (Fortsetzung)

| Bsp. Nr. | $R^1$ | $R^2$ | $R^3$ | X | $R^4$ | $R^6$ | $R^5$ | Fp/°C; $n_D^{20}$ |
|---|---|---|---|---|---|---|---|---|
| 156 | H | H | $CH_3$ | S | H | H | 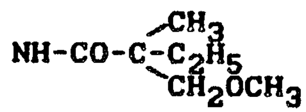 | 125-127 |
| 157 | $-(CH_2)_2-OCH_3$ | H | $-CH_3$ | S | H | H | $-CONHC_4H_9-t$ | 120 |
| 158 | $-(CH_2)_3-OCH_3$ | H | $-CH_3$ | S | H | H | $-CONHC_4H_9-t$ | Öl |
| 159 | $-(CH_2)_2-OC_2H_5$ | H | $-CH_3$ | S | H | H | $-CONHC_4H_9-t$ | Öl |
| 160 | $-(CH_2)_2-OCH_3$ | H | $-CH_3$ | S | H | H | $-CH_3$ | 68 |
| 161 | $-(CH_2)_3-OCH_3$ | H | $-CH_3$ | S | H | H | $-CH_3$ | Öl |
| 162 | $-(CH_2)_2-OC_2H_5$ | H | $-CH_3$ | S | H | H | $-CH_3$ | Öl |
| 163 | 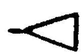 | H | $-CF_3$ | S | H | H | $-CONHC_4H_9-t$ | 80 |

Vorprodukte

Beispiel IIIa-1

4-(3,3,-Dimethylbutyl)-thiophenol

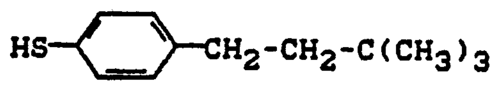

Es werden 7,5 g (0,2 Mol) Natriumborhydrid in 140 ml Diethylenglykoldimethylether vorgelegt und bei 30 - 40° C eine Lösung von 41,6 g (0,2 Mol) 4-(3,3-Dimethylbutyro)-thiophenol in 60 ml Diethylenglykoldimethylether zugetropft. Man läßt 2 Stunden bei 120° C nachreagieren, gibt unter Eiskühlung 100 ml Wasser zu, stellt mit 20 %iger Schwefelsäure sauer und gibt nochmal 200 ml Wasser zu. Die Reaktionsmischung wird nun mit 250 ml Methylenchlorid extrahiert, die organische Phase mit 250 ml Wasser ausgeschüttelt, über Magnesiumsulfat getrocknet und über eine Ringspaltkolonne destilliert. Man erhält 23 g (60 % d. Th.) 4-(3,3-Dimethylbutyl)-thiophenol vom Siedepunkt 98 ° C/0,4 mbar.

Beispiel IIIb-1 (Vorprodukt für Beispiel IIIa-1)

4-(3,3-Diemthylbutyro)-thiophenol

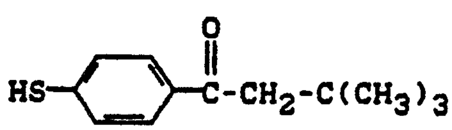

Es werden 18,5 g (0,2 Mol) Natriumhydrogensulfid in 100 ml N-Methylpyrrolidon vorgelegt und durch Andestillieren mit Xylol entwässert. Man läßt auf 140° C abkühlen, gibt 21,05 g (0,1 Mol) 4-(3,3-Dimethylbutyro)-chlorbenzol hinzu, läßt 3 Stunden bei 160° C nachreagieren und destilliert das N-Methylpyrrolidon ab. Der Rückstand wird in 1 Liter Wasser aufgenommen, filtriert und das Filtrat bei 0 - 10° C mit halbkonzentrierter Salzsäure angesäuert. Der Niederschlag wird abgesaugt, neutral gewaschen und getrocknet. Nach Destillation der Mutterlauge unter vermindertem Druck erhält man 14 g (70 % der Theorie) 4-(3,3-Diemthylbutyro)-thiophenol vom Siedepunkt: 110 -115° C/0,1 mbar.

Beispiel XIV-1

2-(4-Nitrophenylthio)-4-hydroxy-6-methylpyrimidin

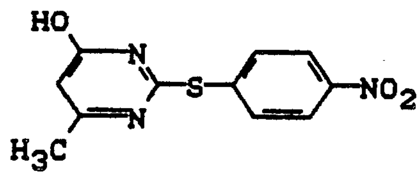

28,4 g (0,2 Mol) 2-Thio-4-hydroxy-6-methylpyrimidin werden in 160 ml Sulfolan suspendiert. Hierzu gibt man bei Raumtemperatur unter Rühren portionsweise 22,4 g (0,4 Mol) pulverisiertes Kaliumhydroxid. Man rührt 30 Minuten nach und gibt anschließend 31,5 g (0,2 Mol) 4-Chlor-nitro-benzol zu. Die Mischung wird 3 Stunden auf 50 °C, dann 3 Stunden auf 120 °C erhitzt, abgekühlt und in 2 Liter Wasser eingerührt. Nach Zugabe von 25 ml konz. Kaliumhydroxid-Lösung wird die Lösung von unlöslichen Bestandteilen filtriert und anschließend mit verdünnter Salzsäure angesäuert. Der Niederschlag wird abgesaugt, mit Wasser neutral gewaschen und bei 80 °C bis 90 °C getrocknet. Man erhält 42,6 g (81 % der Theorie) 2-(4-Nitrophenylthio)-4-hydroxy-6-methylpyrimidin vom Schmelzpunkt Fp: 238 °C bis 240 °C (aus Glykolmonomethylether umkristallisiert).

In analoger Weise gewinnt man:

Beispiel XIV-2:

2-(2-Methoxy-4-nitro-phenylthio)-4-hydroxy-6-methylpyrimidin; Fp: 256 °C bis 258 °C (aus Dimethylformamid).

Beispiel XIV-3:

2-(2-Methyl-4-nitro-phenylthio)-4-hydroxy-6-methyl-pyrimidin; Fp: 212 °C bis 214 °C.

Beispiel IV-1

2-(4-Nitrophenylthio)-4-chlor-6-methylpyrimidin

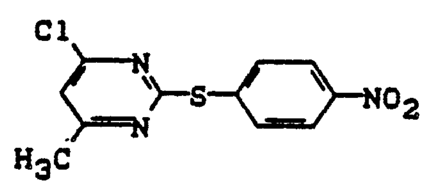

52,6 g (0,2 Mol) 2-(4-Nitro-phenylmercapto)-4-hydroxy-6-methyl-pyrimidin werden in 250 ml Phosphoroxychlorid suspendiert. Die Mischung wird unter Rühren allmählich zum Sieden erhitzt und am Rückfluß gekocht, bis die Chlorwasserstoffentwicklung beendet und eine klare Lösung entstanden ist. Überschüssiges Phosphoroxychlorid wird im Vakuum abdestilliert, der Rückstand in 2 Liter Eiswasser eingetragen. Das abgeschiedene Produkt wird abgesaugt, mit Wasser gewaschen und an der Luft getrocknet. Nach dem Umkristallisieren aus Waschbenzin erhält man 12 g (21,3 % der Theorie) 2-(4-Nitro-phenylthio-)-4-chlor-6-methylpyrimidin vom Schmelzpunkt Fp: 132 °C bis 134 °C.

In analoger Weise wird hergestellt:

Beispiel IV-2:

2-(2-Methoxy-4-nitro-phenylthio)-4-chlor-6-methylpyrimidin; Fp: 140 °C bis 141 °C.

Beispiel IV-3:

2-(2-Methyl-4-nitro-phenylthio)-4-chlor-6-methylpyrimidin; Fp: 118 °C bis 119 °C.

Anwendungsbeispiel

In dem nachfolgenden Anwendungsbeispiel wurde die nachstehend aufgeführte Verbindung als Vergleichssubstanz eingesetzt:

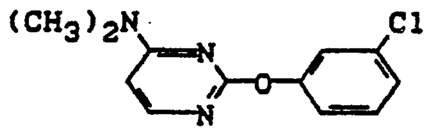

(A)

2-(3-Chlorphenoxy)-4-dimethylaminopyrimidin
(bekannt aus EP-A 1187/Beispiel 28)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testplanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbe handelten Kontrolle.

Es bedeuten:
0 % = keine Wirkung (wie unbehandelte Kontrolle)
100 % = totale Vernichtung

Eine deutliche Überlegenheit in der Nutzpflanzenselektivität wie beispielsweise bei Weizen und in der herbiziden Wirkung gegen mono- und dikotyle Unkräuter wie beispielsweise Amaranthus, Chenopodium, Galinsoga, Sinapis und Setaria gegenüber der Vergleichssubstanz (A) zeigen z.B. die Verbindungen gemäß der Herstellungsbeispiele: 1, 5, 6, 11, 20, 27, 29, 30, 31, 32, 36, 56 und 59.

## Ansprüche

1. Aryloxy (bzw. thio)aminopyrimidine der Formel (I)

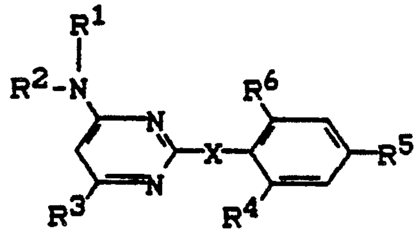

(I)

in welcher
$R^1$ für Wasserstoff, Alkyl, Alkoxyalkyl, Cycloalkyl oder Alkenyl steht,
$R^2$ für Wasserstoff oder Alkyl steht oder
$R^1$ und $R^2$ gemeinsam mit dem Stickstoffatom, an das sie gebunden sind, auch einen drei- bis sechsgliedrigen Ring mit 1 oder 2 weiteren Heteroatomen bilden können,
$R^3$ für Wasserstoff oder gegebenenfalls substituiertes Alkyl steht,
X für Sauerstoff oder Schwefel steht,
$R^4$ und $R^6$ unabhängig voneinander für Wasserstoff, Halogen, Nitro oder für jeweils gegebenenfalls substituiertes Alkyl oder Alkoxy stehen und $R^5$ für Wasserstoff, Halogen, Nitro, Hydroxy, jeweils gegebenenfalls substituiertes Alkyl, Alkenyl oder Alkinyl, jeweils gegebenenfalls substituiertes Alkoxy, Alkoxyalkyloxy, Alkoxyalkoxyalkyloxy, Alkythio, Alkylsulfoxy oder Alkylsulfonyl, jeweils gegebenenfalls substituiertes Alkylcarbonyl oder Alkoxycarbonylalkyl steht oder
$R^5$ für einen Rest der Formel

$-NR^7R^8$

steht, wobei
$R^7$ für Wasserstoff, gegebenenfalls substituiertes Alkyl, für Alkenyl oder Alkinyl steht und
$R^8$ für Wasserstoff, Alkyl oder einen Rest der Formel

- C -R⁹
  ||
  B

steht, in welcher
B für Sauerstoff oder Schwefel steht und
$R^9$ für Alkyl, Alkenyl oder Alkinyl steht, welche gegebenenfalls durch Halogen, Nitro, Cyano, gegebenenfalls substituiertes Aryl, und/oder durch einen oder mehrere Reste der Formel

$-D-R^{10}$

substituiert sind, wobei
D für Sauerstoff, Schwefel, Sulfoxy oder Sulfonyl steht und
$R^{10}$ für Wasserstoff, jeweils gegebenenfalls substituiertes Alkyl, Alkenyl oder Aryl steht, oder
$R^9$ weiterhin für gegebenenfalls substituiertes Cycloalkyl oder einen gegebenenfalls substituierten Heterocyclus steht,
$R^5$ weiterhin für einen Rest der Formeln

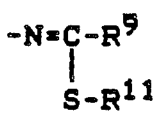

steht wobei
$R^9$ die oben angegebene Bedeutung hat und
$R^{11}$ für gegebenenfalls substituiertes Alkyl, für Alkoxyalkyl, Alkenyl oder Alkinyl steht,

$R^5$ weiterhin für einen Rest der Formel

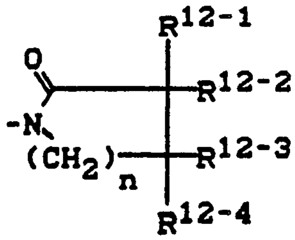

steht, wobei
n für die Zahl 1 oder 2 steht und
$R^{12-1}$, $R^{12-2}$, $R^{12-3}$ und $R^{12-4}$ unabhängig voneinander für Wasserstoff, Halogen oder Alkyl stehen,
$R^5$ weiterhin für einen Rest der Formel

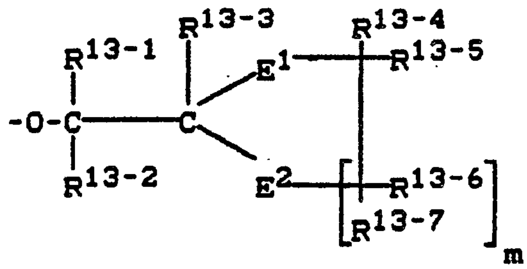

steht, wobei
$E^1$ und $E^2$ unabhängig voneinander für Sauerstoff oder Schwefel stehen und
$R^{13-1}$ bis $R^{13-7}$ unabhängig voneinander für Wasserstoff oder Alkyl stehen und
m für die Zahl 1 oder 2 steht.

2. Aryloxy (bzw. thio)pyrimidine der Formel (I) gemäß Anspruch 1, in welcher
$R^1$ für Wasserstoff, für Alkyl mit 1 bis 6 Kohlenstoffatomen Alkoxyalkyl mit 1 bis 4 Kohlenstoffatomen in den einzelnen Alkylteilen, Cycloalkyl mit 3 bis 6 Kohlenstoffatomen oder Alkenyl mit 3 bis 6 Kohlenstoffatomen steht,
$R^2$ für Wasserstoff oder Alkyl mit 1 bis 6 Kohlenstoffatomen steht oder
$R^1$ und $R^2$ gemeinsam mit dem Stickstoffatom, an das sie gebunden sind, einen drei- bis sechsgliedrigen Ring, der gegebenenfalls 1 oder 2 weitere Heteroatome wie Sauerstoff und/oder Schwefel enthält, bilden können,
$R^3$ für Wasserstoff oder gegebenenfalls durch Halogen, $C_1$-$C_6$-Alkoxy und/oder $C_1$-$C_6$-Alkylthio substituiertes Alkyl mit 1 bis 6 Kohlenstoffatomen steht,
X für Sauerstoff oder Schwefel steht,
$R^4$ und $R^6$ unabhängig voneinander für Wasserstoff, Halogen, Nitro, gegebenenfalls durch Halogen substituiertes Alkyl mit 1 bis 6 Kohlenstoffatomen oder gegebenenfalls durch Halogen substituiertes Alkoxy mit 1 bis 6 Kohlenstoffatomen stehen und
$R^5$ für Wassterstoff, Halogen, Nitro, Hydroxy, gegebenenfalls durch Halogen, $C_1$-$C_6$-Alkoxy und/oder $C_1$-$C_6$-Alkylthio substituiertes Alkyl mit 1 bis 12 Kohlenstoffatomen, gegebenenfalls durch Halogen substituiertes Alkenyl mit 2 bis 12 Kohlenstoffatomen, gegebenenfalls durch Halogen substituiertes Alkinyl mit 2 bis 12 Kohlenstoffatomen, jeweils gegebenenfalls durch Halogen substituiertes Alkoxy, Alkoxyalkyloxy, Alkoxyalkoxyalkyloxy, Alkylthio, Alkylsulfoxy oder Alkylsulfonyl mit jeweils 1 bis 12 Kohlenstoffatomen in den einzelnen Alkylteilen, jeweils gegebenenfalls durch Halogen, $C_1$-$C_6$-Alkoxy und/oder $C_1$-$C_6$-Alkylthio substituiertes Alkylcarbonyl oder Alkoxycarbonylalkyl mit jeweils 1 bis 10 Kohlenstoffatomen in den einzelnen Alkylteilen, für einen Rest der Formel

$-NR^7R^8$

steht, wobei
$R^7$ für Wasserstoff, gegebenenfalls durch Halogen substituiertes Alkyl mit 1 bis 6 Kohlenstoffatomen,

Alkenyl oder Alkinyl mit jeweils 3 bis 6 Kohlenstoffatomen steht und
$R^8$ für Wasserstoff, Alkyl mit 1 bis 10 Kohlenstoffatomen, für einen Rest der Formel

$-C-R^9$
||
B

steht, in welcher
B für Sauerstoff oder Schwefel steht und
$R^9$ für Alkyl mit 1 bis 12 Kohlenstoffatomen, Alkenyl oder Alkinyl mit jeweils 2 bis12 Kohlenstoffatomen steht, wobei Alkyl, Alkenyl und Alkinyl gegebenenfalls substituiert sind durch Halogen, Nitro, Cyano, gegebenenfalls durch Halogen und/ oder Halogen-($C_1$-$C_4$)-alkyl substituiertes Phenyl und/oder durch einen oder mehrere gleiche oder verschiedene Reste der Formel

$-D-R^{10}$

in welcher
D für Sauerstoff, Schwefel, Sulfoxy oder Sulfonyl steht und
$R^{10}$ für Wasserstoff, jeweils gegebenenfalls durch Halogen und/oder $C_1$-$C_6$-Alkoxy substituiertes Alkyl mit 1 bis 6 Kohlenstoffatomen oder Alkenyl mit 2 bis 5 Kohlenstoffatomen, jeweils gegebenenfalls durch Halogen, $C_1$-$C_6$-Alkyl und/oder $C_1$-$C_6$-Alkoxy substituiertes Phenyl oder Naphthyl steht, oder
$R^9$ weiterhin für gegebenenfalls durch Halogen, $C_1$-$C_6$-Alkyl, $C_1$-$C_6$-Alkoxy und/oder Halogen-$C_1$-$C_6$-alkyl substituiertes Cycloalkyl mit 3 bis 8 Kohlenstoffatomen oder einen gegebenenfalls durch $C_1$-$C_6$-Alkyl und/oder $C_1$-$C_6$-Alkoxy substituierten Heterocyclus mit 3 bis 6 Ringgliedern und 1 oder 2 Sauerstoff- und/oder Schwefelatomen steht, oder
$R^5$ weiterhin für einen Rest der Formel

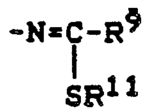

steht, in der
$R^9$ die oben angegebene Bedeutung hat und
$R^{11}$ für gegebenenfalls durch Halogen substituiertes Alkyl mit 1 bis 6 Kohlenstoffatomen, für Alkoxyalkyl mit 1 bis 6 Kohlenstoffatomen je Alkylteil, für Alkenyl oder Alkinyl mit jeweils 3 bis 6 Kohlenstoffatomen steht, oder
$R^5$ für einen Rest der Formel

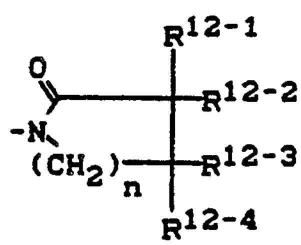

steht, wobei
n für die Zahl 1 oder 2 steht und
$R^{12-1}$, $R^{12-2}$, $R^{12-3}$ und $R^{12-4}$ unabhängig voneinander für Wasserstoff, Halogen oder Alkyl mit 1 bis 6 Kohlenstoffatomen stehen, oder
$R^5$ für einen Rest der Formel

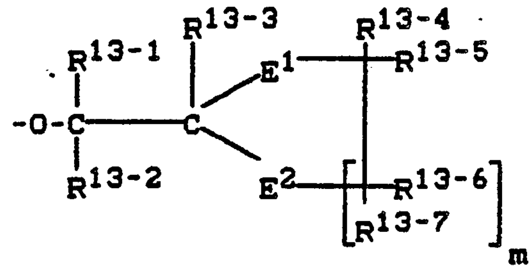

steht, in welcher
$E^1$ und $E^2$ unabhängig voneinander für Sauerstoff oder Schwefel stehen,
$R^{13-1}$ bis $R^{13-7}$ unabhängig voneinander für Wasserstoff oder Alkyl mit 1 bis 6 Kohlenstoffatomen stehen und
m für die Zahl 1 oder 2 steht.

3. Aryloxy (bzw. thio)aminopyrimidine der Formel (I) gemäß Anspruch 1, in welcher $R^1$ für Wasserstoff, Alkyl oder Alkoxyalkyl mit jeweils 1 bis 4 Kohlenstoffatomen in den einzelnen Alkylteilen, Cycloalkyl mit 3 bis 6 Kohlenstoffatomen oder Alkenyl mit 3 bis 4 Kohenstoffatomen steht,
$R^2$ für Wasserstoff oder Alkyl mit 1 bis 4 Kohlenstoffatomen steht oder
$R^1$ und $R^2$ gemeinsam mit dem Stickstoffatom, an das sie gebunden sind, für Aziridino, Pyrrolidino, Piperidino oder Morpholino stehen,
$R^3$ für Wasserstoff oder gegebenenfalls durch Halogen, $C_1$-$C_4$-Alkoxy und/oder $C_1$-$C_4$-Alkylthio substituiertes Alkyl mit 1 bis 4 Kohlenstoffatomen steht,
X für Sauerstoff oder Schwefel steht,
$R^4$ und $R^6$ unabhängig voneinander für Wasserstoff, Fluor, Chlor, Brom, Nitro, gegebenenfalls durch Fluor und/oder Chlor substituiertes Alkyl mit 1 bis 3 Kohlenstoffatomen oder gegebenenfalls durch Fluor und/oder Chlor substituiertes Alkoxy mit 1 bis 3 Kohlenstoffatomen stehen und
$R^5$ für Wasserstoff, Fluor, Chlor, Brom, Nitro, Hydroxy, gegebenenfalls durch Halogen, $C_1$-$C_4$-Alkoxy und/oder $C_1$-$C_4$-Alkylthio substituiertes Alkyl mit 1 bis 10 Kohlenstoffatomen, jeweils gegebenenfalls durch Halogen substituiertes Alkenyl oder Alkinyl mit jeweils 2 bis 9 Kohlenstoffatomen, jeweils gegebenenfalls durch Halogen substituiertes Alkoxy, Alkoxyalkyloxy, Alkoxyalkoxyalkyloxy, Alkylthio, Alkylsulfoxy oder Alkylsulfonyl mit jeweils 1 bis 10 Kohlenstoffatomen, jeweils gegebenenfalls durch Halogen, $C_1$-$C_4$-Alkoxy und/oder $C_1$-$C_4$-Alkylthio substituiertes Alkylcarbonyl oder Alkoxycarbonylalkyl mit jeweils 1 bis 8 Kohlenstoffatomen in den einzelnen Alkylteilen, für einen Rest der Formel

$-NR^7R^8$

steht, wobei
$R^7$ für Wasserstoff, Alkyl mit 1 bis 4 Kohlenstoffatomen, Alkenyl oder Alkinyl mit jeweils 3 bis 4 Kohlenstoffatomen steht und
$R^8$ für Wasserstoff, Alkyl mit 1 bis 8 Kohlenstoffatomen, einen Rest der Formel

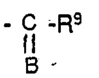

steht, in welcher
B für Sauerstoff oder Schwefel steht und
$R^9$ für Alkyl mit 1 bis 9 Kohlenstoffatomen, Alkenyl oder Alkinyl mit jeweils 2 bis 9 Kohlenstoffatomen steht, wobei Alkyl, Alkenyl und Alkinyl gegebenenfalls substituiert sind durch Halogen, Nitro, Cyano, gegebenenfalls durch Fluor, Chlor, Brom und/oder Halogen-($C_1$-$C_3$)-alkyl substituiertes Phenyl und/oder durch einen, zwei oder drei gleiche oder verschiedene Reste der Formel

$-D-R^{10}$

in welcher
D für Sauerstoff, Schwefel, Sulfoxy oder Sulfonyl steht und
$R^{10}$ für Wasserstoff, jeweils gegebenenfalls durch Halogen und/oder $C_1$-$C_4$-Alkoxy substituiertes Alkyl mit 1 bis 4 Kohlenstoffatomen oder Alkenyl mit 2 bis 4 Kohlenstoffatomen oder jeweils gegebenenfalls durch

Halogen, $C_1$-$C_3$-Alkyl und/oder $C_1$-$C_3$-Alkoxy substituiertes Phenyl oder Naphthyl steht, oder $R^9$ weiterhin für gegebenenfalls durch Halogen, $C_1$-$C_5$-Alkyl, $C_1$-$C_5$-Alkoxy und/oder Halogen-$C_1$-$C_3$-alkyl substituiertes Cycloalkyl mit 3 bis 6 Kohlenstoffatomen oder einen gegebenenfalls durch $C_1$-$C_4$-Alkyl und/oder $C_1$-$C_4$-Alkoxy substituierten Heterocyclus mit 3 bis 6 Ringgliedern und mit 1 oder 2 Sauerstoff- und/oder Schwe felatomen steht und
$R^5$ weiterhin für einen Rest der Formel

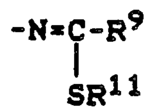

steht, in der
$R^9$ die oben angegebene Bedeutung hat und
$R^{11}$ für gegebenenfalls durch Fluor, Chlor und/oder Brom substituiertes Alkyl mit 1 bis 5 Kohlenstoffatomen, Alkoxyalkyl mit 1 bis 4 Kohlenstoffatomen je Alkylteil, für Alkenyl oder Alkinyl mit jeweils 3 bis 4 Kohlenstoffatomen steht, oder
$R^5$ für einen Rest der Formel

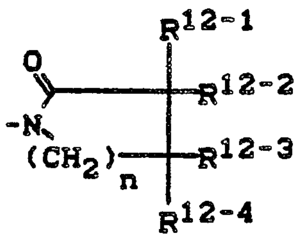

steht, wobei
n für die Zahl 1 oder 2 steht und
$R^{12-1}$, $R^{12-2}$, $R^{12-3}$ und $R^{12-4}$ unabhängig voneinander für Wasserstoff, Fluor, Chlor, Brom oder Alkyl mit 1 bis 4 Kohlenstoffatomen stehen, oder
$R^5$ für einen Rest der Formel

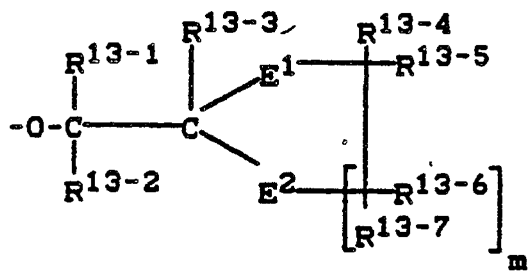

steht, in welcher
$E^1$ und $E^2$ unabhängig voneinander für Sauerstoff oder Schwefel stehen,
$R^{13-1}$ bis $R^{13-7}$ unabhängig voneinander für Wasserstoff oder Alkyl mit 1 bis 6 Kohlenstoffatomen stehen und
m für die Zahl 1 oder 2 steht.

4. Verfahren zur Herstellung von Aryloxy (bzw. thio)aminopyrimidine der Formel (I)

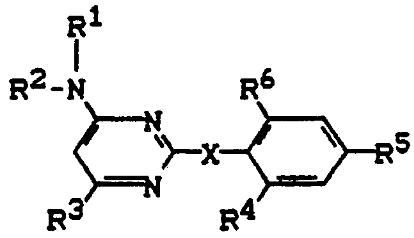

(I)

in welcher
$R^1$ für Wasserstoff, Alkyl, Alkoxyalkyl, Cycloalkyl oder Alkenyl steht,
$R^2$ für Wasserstoff oder Alkyl steht oder
$R^1$ und $R^2$ gemeinsam mit dem Stickstoffatom, an das sie gebunden sind, auch einen drei- bis sechsgliedrigen Ring mit 1 oder 2 weiteren Heteroatomen bilden können,
$R^3$ für Wasserstoff oder gegebenenfalls substituiertes Alkyl steht,
X für Sauerstoff oder Schwefel steht,
$R^4$ und $R^6$ unabhängig voneinander für Wasserstoff, Halogen, Nitro oder für jeweils gegebenenfalls substituiertes Alkyl oder Alkoxy stehen und
$R^5$ für Wasserstoff, Halogen, Nitro, Hydroxy, jeweils gegebenenfalls substituiertes Alkyl, Alkenyl oder Alkinyl, jeweils gegebenenfalls substituiertes Alkoxy, Alkoxyalkyloxy, Alkoxyalkoxyalkyloxy, Alkylthio, Alkylsulfoxy oder Alkylsulfonyl, jeweils gegebenenfalls substituiertes Alkylcarbonyl oder Alkoxycarbonylalkyl steht oder
$R^5$ für einen Rest der Formel

$-NR^7R^8$

steht, wobei
$R^7$ für Wasserstoff, gegebenenfalls substituiertes Alkyl, für Alkenyl oder Alkinyl steht und
$R^8$ für Wasserstoff, Alkyl oder einen Rest der Formel

$$-\underset{\underset{B}{\|}}{C}-R^9$$

steht, in welcher
B für Sauerstoff oder Schwefel steht und
$R^9$ für Alkyl, Alkenyl oder Alkinyl steht, welche gegebenenfalls durch Halogen, Nitro, Cyano, gegebenenfalls substituiertes Aryl, und/oder durch einen oder mehrere Reste der Formel

$-D-R^{10}$

substituiert sind, wobei
D für Sauerstoff, Schwefel, Sulfoxy oder Sulfonyl steht und
$R^{10}$ für Wasserstoff, jeweils gegebenenfalls substituiertes Alkyl, Alkenyl oder Aryl steht, oder
$R^9$ weiterhin für gegebenenfalls substituiertes Cycloalkyl oder einen gegebenenfalls substituierten Heterocyclus steht,
$R^5$ weiterhin für einen Rest der Formeln

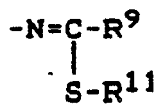

steht, wobei
$R^9$ die oben angegebene Bedeutung hat und
$R^{11}$ für gegebenenfalls durch substituiertes Alkyl, für Alkoxyalkyl, Alkenyl oder Alkinyl steht,
$R^5$ für einen Rest der Formel

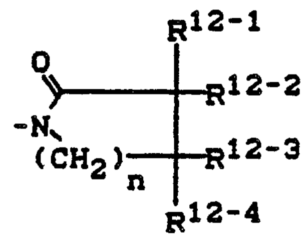

steht, wobei
n für die Zahl 1 oder 2 steht und
$R^{12-1}$, $R^{12-2}$, $R^{12-3}$ und $R^{12-4}$ unabhängig voneinander für Wasserstoff, Halogen oder Alkyl stehen,
$R^5$ für einen Rest der Formel

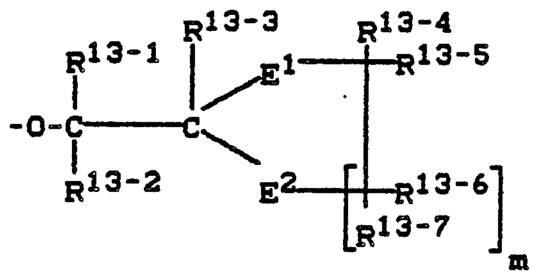

steht, wobei
$E^1$ und $E^2$ unabhängig voneinander für Sauerstoff oder Schwefel stehen und
$R^{13-1}$ bis $R^{13-7}$ unabhängig voneinander für Wasserstoff oder Alkyl stehen und
m für die Zahl 1 oder 2 steht.
dadurch gekennzeichnet, daß man
A) Pyrimidinderivate der Formel (II),

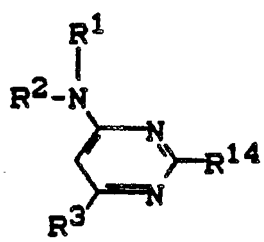

(II)

in welcher
$R^1$, $R^2$ und $R^3$ die oben angegebene Bedeutung haben und
$R^{14}$ für Halogen oder Alkylsulfonyl steht,
mit (Thio)phenolen der Formel (III)

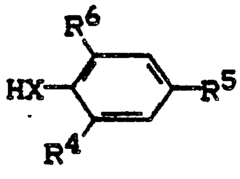

(III)

in welcher
$R^4$, $R^5$, $R^6$ und X die oben angegebene Bedeutung haben
gegebenenfalls in Gegenwart eines Verdünnungsmittels und gegebenenfalls in Gegenwart eines Säurebindemittels umsetzt, oder daß man
B) Halogenpyrimidinderivate der allgemeinen Formel (IV)

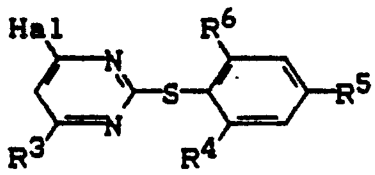

(IV)

in welcher
$R^3$, $R^4$, $R^5$ und $R^6$ die oben angegebene Bedeutung haben mit der Maßgabe, daß mindestens einer der Reste $R^4$, $R^5$ oder $R^6$ starke Elektronenakzeptoreigenschaft besitzt, und
Hal für Halogen steht,
mit einem Amin der allgemeinen Formel (V)

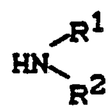

(V)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt oder daß man

C-$C_1$) Aryloxy (bzw. thio)aminopyrimidine der Formel (Ia)

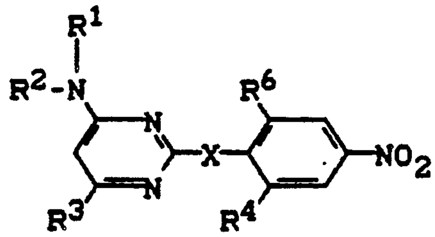

(Ia)

in welcher
$R^1$, $R^2$, $R^3$, $R^4$, $R^6$ und X die oben angegebene Bedeutung haben,
mit Reduktionsmitteln gegebenenfalls in Gegenwart eines Verdünnungsmittels und gegebenenfalls in Gegenwart eines Katalysators umsetzt und daß man

C-$C_2$) die nach Verfahren C-$C_1$ erhaltenen Aryloxy (bzw. thio)aminopyrimidine der Formel (Ib)

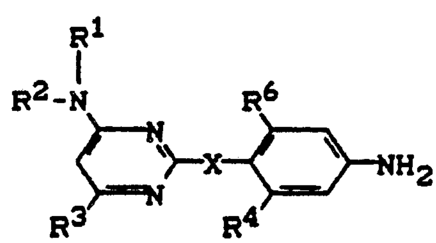

(Ib)

in welcher
$R^1$, $R^2$, $R^3$, $R^4$, $R^6$ und X die oben angegebene Bedeutung haben,
α) mit Säurehalogeniden der Formel (VI),

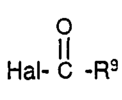

(VI)

in welcher

$R^9$ die oben angegebene Bedeutung hat und
Hal für Halogen, insbesondere Chlor oder Brom steht,
gegebenenfalls in Gegenwart eines Verdünnungsmittels und gegebenenfalls in Gegenwart eines Säurebindemittels umsetzt,
oder
β) mit symmetrischen Carbonsäureanhydriden der allgemeinen Formel (VII),

$$R^9-\overset{O}{\overset{||}{C}}-O-\overset{O}{\overset{||}{C}}-R^9 \qquad (VII)$$

in welcher
$R^9$ die oben angegebene Bedeutung hat,
gegebenenfalls in Gegenwart eines Verdünnungsmittels umsetzt,
oder
γ) mit asymmetrischen Säureanhydriden der Formel (VIII),

$$R-O-\overset{O}{\overset{||}{C}}-O-\overset{O}{\overset{||}{C}}-R^9 \qquad (VIII)$$

in welcher
$R^9$ die oben angegebene Bedeutung hat und
R für Alkyl oder Alkylsulfonyl mit jeweils 1 bis 4 Kohlenstoffatomen, Phenyl oder Phenylsulfonyl steht,
gegebenenfalls in Gegenwart eines Verdünnungsmittels umsetzt,
oder
δ) mit ω-Halogencarbonsäurehalogeniden der Formel (IX),

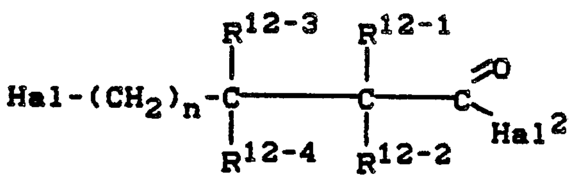

(IX)

in welcher
$R^{12-1}$, $R^{12-2}$, $R^{12-3}$, $R^{12-4}$ und n die oben angegebene Bedeutung haben und
Hal und $Hal^2$ für Halogen stehen,
gegebenenfalls in Gegenwart eines Säurebindemittels und gegebenenfalls in Gegenwart eines Verdünnungsmittels in einer zweistufigen Reaktionsfolge umsetzt,
oder daß man
D) Aryloxy (bzw. thio)aminopyrimidine der Formel (Ic)

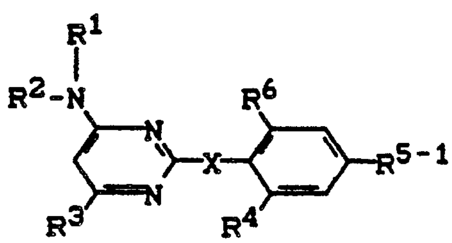

(Ic)

in welcher
$R^1$, $R^2$, $R^3$, $R^4$, $R^6$ und X die oben angegebene Bedeutung haben, und
$R^{5-1}$ für den Rest

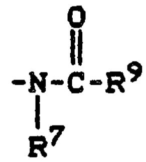

steht, wobei $R^7$ und $R^9$ die oben angegebene Bedeutung haben, mit Schwefelungsreagenzien, umsetzt zu Verbindungen der Formel (Id)

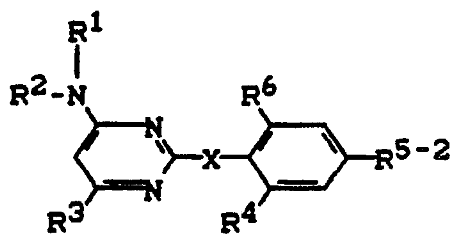

(Id)

in welcher
$R^1$, $R^2$, $R^3$, $R^4$, $R^6$ und X die oben angegebene Bedeutung haben, und
$R^{5-2}$ für den Rest

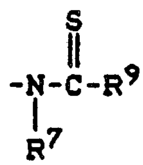

steht, wobei $R^7$ und $R^9$ die oben angegebene Bedeutung haben,
oder daß man

E) die nach dem Verfahren D) hergestellten Verbindungen (Id), in denen $R^7$ für Wasserstoff steht, mit Verbindungen der Formel (X)

$L-R^{11}$ (X)

in welcher
$R^{11}$ die oben angegebene Bedeutung hat und
L für Halogen, Alkylsulfonyloxy oder gegebenenfalls substituiertes Phenylsulfonyloxy steht,
mit deprotonierenden Basen und gegebenenfalls in Gegenwart eines Verdünnungsmittels zu Verbindungen der allgemeinen Formel (Ie) umsetzt

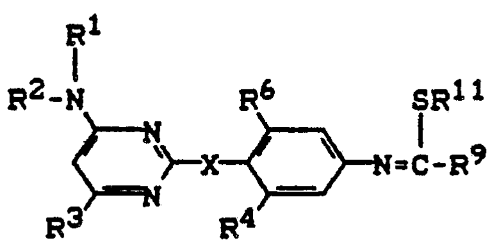

(Ie)

in welcher
$R^1$, $R^2$, $R^3$, $R^4$, $R^6$, $R^9$, $R^{11}$ und X die oben angegebene Bedeutung haben,
oder daß man

F) Aryloxy (bzw. thio)aminopyrimidine der Formel (If)

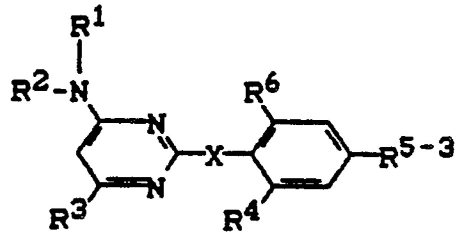

(If)

in welcher
$R^1$, $R^2$, $R^3$, $R^4$, $R^6$ und X die oben angegebene Bedeutung haben, und
$R^{5-3}$ für Hydroxy steht,
mit Verbindungen der allgemeinen Formel (XI),

$R^{5-4}$-G (XI)

in welcher
$R^{5-4}$ für jeweils gegebenenfalls substituiertes Alkyl, Alkenyl, Alkoxyalkyl, Alkylthioalkyl oder Alkylcarbonylalkyl oder für einen Rest der Formel

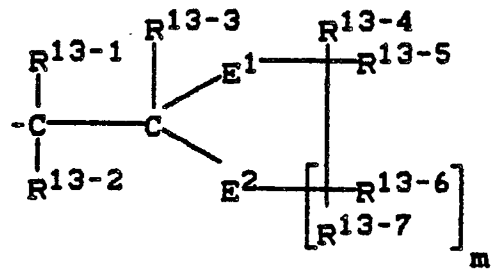

steht, wobei
$E^1$, $E^2$, $R^{13-1}$ bis $R^{13-7}$ und m die oben angegebene Bedeutung haben, und
G für Halogen, Alkylsulfonyloxy oder gegebenenfalls substituiertes Phenylsulfonyloxy steht,
in Gegenwart eines Verdünnungsmittels und gegebenenfalls in Gegenwart eines Säurebindemittels umsetzt.

5. Herbizide Mittel, gekennzeichnet durch einen Gehalt an mindestens einem Aryloxy (bzw. thio)-aminopyrimidin der Formel (I) gemäß den Ansprüchen 1 bis 4.

6. Verfahren zur Bekämpfung von Unkräutern, dadurch gekennzeichnet, daß man Aryloxy (bzw. thio)-aminopyrimidine der Formel (I) gemäß den Ansprüchen 1 bis 4 auf die Unkräuter und/oder ihren Lebensraum einwirken läßt.

7. Verwendung von Aryloxy (bzw. thio)aminopyrimidinen der Formel (I) gemäß den Ansprüchen 1 bis 4 zur Bekämpfung von Unkräutern.

8. Verfahren zur Herstellung von herbiziden Mitteln, dadurch gekennzeichnet, daß man Aryloxy (bzw. thio)aminopyrimidine der Formel (I) gemäß den Ansprüchen 1 bis 4 mit Streckmitteln und/oder oberflächenaktiven Substanzen vermischt.

9. Thiophenole der Formel (IIIa)

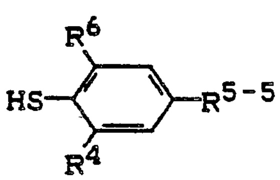

(IIIa)

in welcher
$R^4$ und $R^6$ unabhängig voneinander für Wasserstoff, Halogen, Nitro oder für jeweils gegebenenfalls substituiertes Alkyl oder Alkoxy stehen und
$R^{5-5}$ für gegebenenfalls substituiertes Alkyl mit mindestens einem tertiären Kohlenstoffatom steht, mit der

Maßgabe, daß $R^{5-5}$ nicht für tert.-Butyl steht, wenn $R^4$ und $R^6$ gleichzeitig für Wasserstoff stehen.

10. Verfahren zur Herstellung von Thiophenolen der Formel (IIIa)

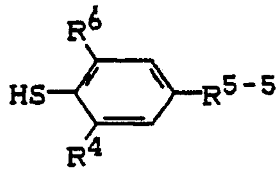

(IIIa)

in welcher

$R^4$ und $R^6$ unabhängig voneinander für Wasserstoff, Halogen, Nitro oder für jeweils gegebenenfalls substituiertes Alkyl oder Alkoxy stehen und

$R^{5-5}$ für gegebenenfalls substituiertes Alkyl mit mindestens einem tertiären Kohlenstoffatom steht, mit der Maßgabe, daß $R^{5-5}$ nicht für tert.-Butyl steht, wenn $R^4$ und $R^6$ gleichzeitig für Wasserstoff stehen,

dadurch gekennzeichnet, daß man Verbindungen der Formel (IIIb)

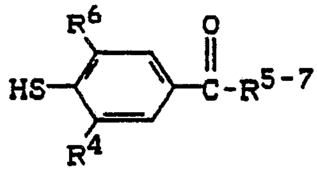

(IIIb)

in welcher

$R^4$ und $R^6$ die oben angegebene Bedeutung haben und

$R^{5-7}$ für gegebenenfalls substituiertes Alkyl mit mindestens einem tertiären Kohlenstoffatom steht, mit Reduktionsmitteln in Gegenwart eines Verdünnungsmittels umsetzt.